(19) Europäisches Patentamt
European Patent Office
Office européen des brevets

(11) EP 1 544 308 B1

(12) EUROPEAN PATENT SPECIFICATION

(45) Date of publication and mention of the grant of the patent:
**28.01.2009 Bulletin 2009/05**

(51) Int Cl.:
***C12Q 1/68*** *(2006.01)*

(21) Application number: **03293159.4**

(22) Date of filing: **15.12.2003**

(54) **Repertoire determination of a lymphocyte B population**

Ermittlung des Repertoires von B-Lymphozyten Populationen

Détermination du répertoire d'une population de lymphocytes B

(84) Designated Contracting States:
**AT BE BG CH CY CZ DE DK EE ES FI FR GB GR HU IE IT LI LU MC NL PT RO SE SI SK TR**

(43) Date of publication of application:
**22.06.2005 Bulletin 2005/25**

(73) Proprietors:
- **INSTITUT PASTEUR**
  **75015 Paris (FR)**
- **INSTITUT NATIONAL DE LA SANTE ET DE LA RECHERCHE MEDICALE (INSERM)**
  **75013 Paris (FR)**

(72) Inventors:
- **Lim, Annick**
  **92120 Montrouge (FR)**
- **Lemercier, Brigitte**
  **92320 Chatillon (FR)**
- **Kourilsky, Philippe**
  **75001 Paris (FR)**
- **Huetz, François**
  **75013 Paris (FR)**

(74) Representative: **Ahner, Francis et al**
**Cabinet Régimbeau**
**20, rue de Chazelles**
**75847 Paris cedex 17 (FR)**

(56) References cited:
- **FEUCHTENBERGER M ET AL: "Semiquantitative and qualitative assessment of B-lymphocyte VH repertoire by a fluorescent multiplex PCR" JOURNAL OF IMMUNOLOGICAL METHODS, ELSEVIER SCIENCE PUBLISHERS B.V., AMSTERDAM, NL, vol. 276, no. 1-2, 1 May 2003 (2003-05-01), pages 121-127, XP004422646 ISSN: 0022-1759**
- **DELASSUS S ET AL: "PCR-based analysis of the murine immunoglobulin heavy-chain repertoire" JOURNAL OF IMMUNOLOGICAL METHODS, ELSEVIER SCIENCE PUBLISHERS B.V., AMSTERDAM, NL, vol. 184, no. 2, 18 August 1995 (1995-08-18), pages 219-229, XP004020989 ISSN: 0022-1759**
- **LIM A ET AL: "Combination of MHC-peptide multimer-based T cell sorting with the Immunoscope permits sensitive ex vivo quantitation and follow-up of human CD8<+> T cell immune responses" JOURNAL OF IMMUNOLOGICAL METHODS, ELSEVIER SCIENCE PUBLISHERS B.V.,AMSTERDAM, NL, vol. 261, no. 1-2, 1 March 2002 (2002-03-01), pages 177-194, XP004341278 ISSN: 0022-1759**
- **COLLETTE A ET AL: "New methods and software tools for high throughput CDR3 spectratyping. Application to T lymphocyte repertoire modifications during experimental malaria" JOURNAL OF IMMUNOLOGICAL METHODS, ELSEVIER SCIENCE PUBLISHERS B.V., AMSTERDAM, NL, vol. 278, no. 1-2, July 2003 (2003-07), pages 105-116, XP004453168 ISSN: 0022-1759**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 544 308 B1

- PANNETIER C ET AL: "THE SIZES OF THE CDR3 HYPERVARIABLE REGIONS OF THE MURINE T-CELL RECEPTOR BETA CHAINS VARY AS A FUNCTION OF THE RECOMBINED GERM-LINE SEGMENTS" PROCEEDINGS OF THE NATIONAL ACADEMY OF SCIENCES OF USA, NATIONAL ACADEMY OF SCIENCE. WASHINGTON, US, vol. 90, May 1993 (1993-05), pages 4319-4323, XP002951502 ISSN: 0027-8424
- ARSTILA T PETTERI ET AL: "A direct estimate of the human alphabeta T cell receptor diversity" SCIENCE (WASHINGTON D C), vol. 286, no. 5441, 29 October 1999 (1999-10-29), pages 958-961, XP001191021 ISSN: 0036-8075
- LEFRANC M P: "IMGT, the international ImMunoGeneTics database." NUCLEIC ACIDS RESEARCH. 1 JAN 2001, vol. 29, no. 1, 1 January 2001 (2001-01-01), pages 207-209, XP002281078 ISSN: 1362-4962
- DATABASE EBI [Online] EMBL; Sequence 56 from US6300129 16 September 2003 (2003-09-16), LONBERG, N., KAY, R.M.: XP002281108 retrieved from EMBL accession no. AR369855 Database accession no. AR369855
- DATABASE EBI EMBL; Sequence 7 from WO0175091 29 October 2001 (2001-10-29), OHLIN, M. ET AL.: XP002281109 retrieved from EMBL accession no. AX268890 Database accession no. AX268890 & WO 01/75091 A (BIOINVENT INTERNAT AB ; CARLSSON ROLAND (SE); OHLIN MATS (SE); SODERLI) 11 October 2001 (2001-10-11)
- DATABASE EBI EMBL; Sequence 18 from WO0175091 29 October 2001 (2001-10-29), OHLIN, M. ET AL.: XP002281110 retrieved from EMBL accession no. AX268901 Database accession no. AX268901 & WO 01/75091 A (BIOINVENT INTERNAT AB ; CARLSSON ROLAND (SE); OHLIN MATS (SE); SODERLI) 11 October 2001 (2001-10-11)
- DATABASE EBI EMBL; Sequence 21 of US6005091 13 September 2000 (2000-09-13), BLACKBURN M. NEAL ET AL.: XP002281111 retrieved from EMBL accession no. AR096134 Database accession no. AR096134
- DATABASE EBI EMBL; Sequence 146 from US6300129 16 September 2003 (2003-09-16), LONBERG, N. ET AL.: XP002281112 retrieved from EMBL accession no. AR369914 Database accession no. AR369914
- DATABASE EBI EMBL; Sequence 350 from US6300129 16 September 2003 (2003-09-16), LONBERG N. ET AL.: XP002281113 retrieved from EMBL accession no. AR370100 Database accession no. AR370100
- DATABASE EBI EMBL; Sequence 2 from US2002168397 11 April 2003 (2003-04-11), TSUJI, K. ET AL.: XP002281114 retrieved from EMBL accession no. AR264699 Database accession no. AR264699
- DATABASE EBI EMBL; Sequence 4 from WO8803145 16 February 1995 (1995-02-16), LIU, A.Y. ET AL.: XP002281115 retrieved from EMBL accession no. I05932 Database accession no. I05932 & WO 88/03145 A (ONCOGEN ; INT GENETIC ENG (US)) 5 May 1988 (1988-05-05)
- DATABASE EBI EMBL; Sequence 10 from WO0213862 9 August 2002 (2002-08-09), GOLD, D.P. ET AL.: XP002281116 retrieved from EMBL accession no. AX473360 Database accession no. AX473360 & WO 02/13862 A (GOLD DANIEL P ; FAVRILLE INC (US); SHOPES ROBERT J (US)) 21 February 2002 (2002-02-21)
- DATABASE EBI EMBL; Sequence 36 from WO9915696 26 January 2000 (2000-01-26), QIN, Y.: XP002281117 retrieved from EMBL accession no. A97536 Database accession no. A97536 & WO 99/15696 A (QIN YUFEN) 1 April 1999 (1999-04-01)
- DATABASE EBI EMBL; JH reporter probe from WO9219775 24 November 1994 (1994-11-24), XP002281118 retrieved from EMBL accession no. A22452 Database accession no. A22452 & WO 92/19775 A (RAGGIO ITALGENE SPA) 12 November 1992 (1992-11-12)
- WHITE HARRY N: "Restriction-PCR fingerprinting of the immunoglobulin VH repertoire: Direct detection of an immune response and global analysis of B cell clonality" EUROPEAN JOURNAL OF IMMUNOLOGY, vol. 28, no. 10, October 1998 (1998-10), pages 3268-3279, XP002281079 ISSN: 0014-2980
- FOREMAN A.L. ET AL: 'VH gene usage and CDR3 analysis of B cell receptor in the peripheral blood of patients with PBC' AUTOIMMUNITY vol. 41, no. 1, February 2008, pages 80 - 86
- LIM A. ET AL: 'The IgE repertoire in PBMCs of atopic patients is characterized by individual rearrangements without variable region of the heavy immunoglobulin chain bias' BASIC AND CLINICAL IMMUNOLOGY vol. 120, September 2007, pages 696 - 706
- BELLONI B. ET AL: 'Low-dose anti-IgE therapy in patients with atopic eczema with high serum IgE levels' JOURNAL OF ALLERGY AN CLINICAL IMMUNOLOGY vol. 120, 15 October 2007, pages 1223 - 1225

SEQUENCE LISTING

<110> Institut Pasteur Centre National de la Recherche Scientifique (CNRS) Institut National de la Santé et de la Recherche Médicale (INSERM)

<120> Repertoire determination of a lymphocyte B population

<130> D21747

<160> 39

<170> PatentIn version 3.2

<210> 1
<211> 21
<212> DNA
<213> Artificial

<220>
<221> source
<222> (1)..(21)
<223> /note="description of artificial sequence: Forward primer HUMVH1a specific for the nucleic acid encoding a VH segment of the VH1 subgroup"

<400> 1
agtgaaggtc tcctgcaagg c 21

<210> 2
<211> 21
<212> DNA
<213> Artificial

<220>
<221> source
<222> (1) .. (21)
<223> /note="description of artificial sequence: Forward primer HUMVH1b specific for the nucleic acid encoding a VH segment of the VH1 subgroup"

<400> 2
agtgaaggtt tcctgcaagg c 21

<210> 3
<211> 21
<212> DNA
<213> Artificial

<220>
<221> source
<222> (1) .. (21)
<223> /note="description of artificial sequence: Forward primer HUMVH1c specific for the nucleic acid encoding a VH segment of the VH1 subgroup"

<400> 3
agtgaarrtc tcctgcaagg t 21

<210> 4

<211> 19
<212> DNA
<213> Artificial

<220>
<221> source
<222> (1)..(19)
<223> /note="description of artificial sequence: Forward primer HUMVH2 specific for the nucleic acid encoding a VH segment of the VH2 subgroup"

<400> 4
aacccacasa gaccctcac 19

<210> 5
<211> 24
<212> DNA
<213> Artificial

<220>
<221> source
<222> (1)..(24)
<223> /note="description of artificial sequence: Forward primer HUMVH3aa specific for the nucleic acid encoding a VH segment of the VH3a subgroup"

<400> 5
gcagattcac catctcaaga gatg 24

<210> 6
<211> 24
<212> DNA
<213> Artificial

<220>
<221> source
<222> (1)..(24)
<223> /note="description of artificial sequence: Forward primer HUMVH3ab specific for the nucleic acid encoding a VH segment of the VH3a subgroup"

<400> 6
gcaggttcac catctccaga gatg 24

<210> 7
<211> 22
<212> DNA
<213> Artificial

<220>
<221> source
<222> (1)..(22)
<223> /note="description of artificial sequence: Forward primer HUMVH3ba specific for the nucleic acid encoding a VH segment of the VH3b subgroup"

<400> 7
gccgattcac catctccaga ga 22

<210> 8
<211> 22
<212> DNA

<213> Artificial

<220>
<221> source
<222> (1)..(22)
<223> /note="description of artificial sequence: Forward primer HUMVH3bb specific for the nucleic acid encoding a VH segment of the VH3b subgroup"

<400> 8
gcagattcac catctccaga ga 22

<210> 9
<211> 22
<212> DNA
<213> Artificial

<220>
<221> source
<222> (1)..(22)
<223> /note="description of artificial sequence: Forward primer HUMVH3bc specific for the nucleic acid encoding a VH segment of the VH3b subgroup"

<400> 9
gccgattcac catctccagg ga 22

<210> 10
<211> 22
<212> DNA
<213> Artificial

<220>
<221> source
<222> (1)..(22)
<223> /note="description of artificial sequence: Forward primer HUMVH3bd specific for the nucleic acid encoding a VH segment of the VH3b subgroup"

<400> 10
gcaggttcac catctccaga ga 22

<210> 11
<211> 22
<212> DNA
<213> Artificial

<220>
<221> source
<222> (1) .. (22)
<223> /note="description of artificial sequence: Forward primer HUMVH4a specific for the nucleic acid encoding a VH segment of the VH4 subgroup"

<400> 11
ctacaacccg tccctcaaga gt 22

<210> 12
<211> 22
<212> DNA
<213> Artificial

<220>
<221> source
<222> (1) .. (22)
<223> /note="description of artificial sequence: Forward primer HUMVH4b specific for the nucleic acid encoding a VH segment of the VH4 subgroup"

<400> 12
ctacaacccc tccctcaaga gt 22

<210> 13
<211> 18
<212> DNA
<213> Artificial

<220>
<221> source
<222> (1) .. (18)
<223> /note="description of artificial sequence: Forward primer HUMVH5 specific for the nucleic acid encoding a VH segment of the VH5 subgroup"

<400> 13
gtgaaaaagc ccggggag 18

<210> 14
<211> 18
<212> DNA
<213> Artificial

<220>
<221> source
<222> (1) .. (18)
<223> /note="description of artificial sequence: Forward primer HUMVH6 specific for the nucleic acid encoding a VH segment of the VH6 subgroup"

<400> 14
tccggggaca gtgtctct 18

<210> 15
<211> 21
<212> DNA
<213> Artificial

<220>
<221> source
<222> (1) .. (21)
<223> /note="description of artificial sequence: Forward primer HUMVH7 specific for the nucleic acid encoding a VH segment of the VH7 subgroup"

<400> 15
ggtgcaatct gggtctgagt t 21

<210> 16
<211> 17
<212> DNA
<213> Artificial

<220>
<221> source

<222> (1)..(17)
<223> /note="description of artificial sequence: Reverse primer IGJH1 specific for the nucleic acid encoding a JH segment of the JH1 subgroup"

<400> 16
ccctggcccc agtgctg 17

<210> 17
<211> 18
<212> DNA
<213> Artificial

<220>
<221> source
<222> (1)..(18)
<223> /note="description of artificial sequence: Reverse primer IGJH2 specific for the nucleic acid encoding a JH segment of the JH2 subgroup"

<400> 17
ccacggcccc agagatcg 18

<210> 18
<211> 23
<212> DNA
<213> Artificial

<220>
<221> source
<222> (1) .. (23)
<223> /note="description of artificial sequence: Reverse primer IGJH3 specific for the nucleic acid encoding a JH segment of the JH3 subgroup"

<400> 18
cccttggccc cagayatcaa aag 23

<210> 19
<211> 19
<212> DNA
<213> Artificial

<220>
<221> source
<222> (1)..(19)
<223> /note="description of artificial sequence: Reverse primer IGJH4.1 specific for the nucleic acid encoding a JH segment of the JH4 subgroup"

<400> 19
ggttccttgg ccccagtag 19

<210> 20
<211> 19
<212> DNA
<213> Artificial

<220>
<221> source
<222> (1)..(19)
<223> /note="description of artificial sequence: Reverse primer IGJH4.2 specific for the nucleic acid encoding a JH segment of the JH4 subgroup"

<400> 20
ggttccctgg ccccagtag 19

<210> 21
<211> 19
<212> DNA
<213> Artificial

<220>
<221> source
<222> (1)..(19)
<223> /note="description of artificial sequence: Reverse primer IGJH4.3 specific for the nucleic acid encoding a JH segment of the JH4 subgroup"

<400> 21
ggtcccttgg ccccagtag 19

<210> 22
<211> 18
<212> DNA
<213> Artificial

<220>
<221> source
<222> (1)..(18)
<223> /note="description of artificial sequence: Reverse primer IGJH5 specific for the nucleic acid encoding a JH segment of the JH5 subgroup"

<400> 22
tggccccagg rgtcgaac 18

<210> 23
<211> 20
<212> DNA
<213> Artificial

<220>
<221> source
<222> (1) .. (20)
<223> /note="description of artificial sequence: Reverse primer IGJH6.1 specific for the nucleic acid encoding a JH segment of the JH6 subgroup"

<400> 23
ccttgccccc agacgtccat 20

<210> 24
<211> 20
<212> DNA
<213> Artificial

<220>
<221> source
<222> (1)..(20)
<223> /note="description of artificial sequence: Reverse primer IGJH6.2 specific for the nucleic acid encoding a JH segment of the JH6 subgroup"

<400> 24
ccttggcccc agacgtccat 20

<210> 25
<211> 20
<212> DNA

<213> Artificial

<220>
<221> source
<222> (1)..(20)
<223> /note="description of artificial sequence: Reverse primer IGJH6.3 specific for the nucleic acid encoding a JH segment of the JH6 subgroup"

<400> 25
cctttgcccc agacgtccat 20

<210> 26
<211> 16
<212> DNA
<213> Artificial

<220>
<221> source
<222> (1)..(16)
<223> /note="description of artificial sequence: Reverse primer HIGCM specific for the nucleic acid encoding a CH segment of the IgM heavy chain"

<400> 26
cagccaacgg ccacgc 16

<210> 27
<211> 19
<212> DNA
<213> Artificial

<220>
<221> source
<222> (1)..(19)
<223> /note="description of artificial sequence: Reverse primer HIGCGa specific for the nucleic acid encoding a CH segment of the IgG heavy chain"

<400> 27
tcagagcgcc tgagttcca 19

<210> 28
<211> 19
<212> DNA
<213> Artificial

<220>
<221> source
<222> (1)..(19)
<223> /note="description of artificial sequence: Reverse primer HIGCGb specific for the nucleic acid encoding a CH segment of the IgG heavy chain"

<400> 28

tcagggcgcc tgagttcca 19

<210> 29
<211> 15
<212> DNA
<213> Artificial

<220>
<221> source
<222> (1)..(15)
<223> /note="description of artificial sequence: Hydrolysis probe HCM specific for the nucleic acid encoding the CH segment of the IgM heavy chain "

<400> 29
ccgtcggata cgagc 15

<210> 30
<211> 19
<212> DNA
<213> Artificial

<220>
<221> source
<222> (1) .. (19)
<223> /note="description of artificial sequence: Reverse probe HCM specific for the nucleic acid encoding the CH segment of the IgM heavy chain"

<400> 30
ggagacgagg gggaaaagg 19

<210> 31
<211> 18
<212> DNA
<213> Artificial

<220>
<221> source
<222> (1) .. (18)
<223> /note="description of artificial sequence: VH5 internal forward primer specific for the nucleic acid encoding a VH segment of the VH5 subgroup"

<400> 31
agcccgggga gtctctga 18

<210> 32
<211> 17
<212> DNA
<213> Artificial

<220>
<221> source
<222> (1)..(17)
<223> /note="description of artificial sequence: Hydrolysis probe specific for the nucleic acid encoding a VH segment of the VH5 subgroup"

<400> 32
acccttacag gagatct 17

<210> 33
<211> 20
<212> DNA
<213> Artificial

<220>
<221> source
<222> (1) .. (20)
<223> /note="description of artificial sequence: CH reverse primer HIGCE specific for the nucleic acid encoding a CH segment of the IgE"

<400> 33
tcacggaggt ggcattggag 20

<210> 34
<211> 13
<212> DNA
<213> Artificial

<220>
<221> source
<222> (1)..(13)
<223> /note="description of artificial sequence: CH reverse hydrolysis probe HCG specific for the nucleic acid encoding a CH segment of the IgG heavy chain"

<400> 34
ccggtgacgg tgc 13

<210> 35
<211> 22
<212> DNA
<213> Artificial

<220>
<221> source
<222> (1) .. (22)
<223> /note="description of artificial sequence: CH reverse probe HCG specific for the nucleic acid encoding a CH segment of the IgG heavy chain"

<400> 35
aagtagtcct tgaccaggca gc 22

<210> 36
<211> 16
<212> DNA
<213> Artificial

<220>
<221> source
<222> (1)..(16)
<223> /note="description of artificial sequence: CH reverse hydrolysis probe specific for the nucleic acid encoding a CH segment of the IgE"

<400> 36
tgctgcaaaa acattc 16

<210> 37
<211> 19

<212> DNA
<213> Artificial

<220>
<221> source
<222> (1)..(19)
<223> /note="description of artificial sequence: CH reverse probe specific for the nucleic acid encoding a CH segment of the IgE"

<400> 37
cgggtcaagg ggaagacgg 19

<210> 38
<211> 14
<212> PRT
<213> Artificial

<220>
<221> source
<222> (1) .. (14)
<223> /note="description of artificial sequence: Amino acid CDR3 sequence of the clonal expansion A"

<400> 38

```
Thr His Ile Gly Tyr Ser Ala Ala Gly Trp Tyr Phe Asp Leu
1               5                   10
```

<210> 39
<211> 25
<212> PRT
<213> Artificial

<220>
<221> source
<222> (1) .. (25)
<223> /note="description of artificial sequence: /note="description of artificial sequence: Amino acid CDR3 sequence of the clonal expansion B"

<400> 39

```
Leu Gly Tyr Cys Ser Gly Gly Ser Cys Tyr Gly Val Gly Cys Gly Ala
1               5                   10                  15

Asp Cys Tyr Arg Glu Tyr Phe Gln Asp
            20                  25
```

## Description

The present invention provides a process for determining the quantitative and qualitative profile of the repertoire of a given type of an immunoglobulin (Ig) heavy chain expressed by a lymphocyte B population present in a tissue sample, and kits and uses thereof. It also provides a set of VH forward primers associated with a CH reverse primer, which are respectively capable of specifically hybridizing in stringent conditions with the nucleic acids encoding the variable segments of Ig heavy-chains and with the constant segment of a given type of Ig heavy chain, such as preferably IgM, IgG, IgE or IgA heavy chain. Methods for the *in vitro* diagnosis of a condition associated with an abnormal expression of the repertoire of a given type of Ig heavy chain, and for the *in vitro* follow-up of a treatment of such a condition, are also comprised herein.

An essential feature of the immune system is the capacity to recognize specifically a large number of antigens. In vertebrates, the B lymphocytes partly execute this function of recognition, by means of the immunoglobulins.

To the tremendous variety of antigens to be recognized, there corresponds a very wide potential diversity of these immunoglobulins. Indeed, immunoglobulins are composed of four peptide chains, the NH2 terminal domains of which are highly variable. It is the existence of a strong interaction between a given antigenic determinant and the site consisting of these variable domains which constitutes the expression at molecular level of the phenomenon of recognition. Thus, the information contained in the genome of a mouse enables it to produce potentially at least $10^{11}$ immunoglobulins of different variable region.

The notion of repertoire thus emerges: the set of immunoglobulin variable regions present at a given instant in an organism for a given type of immunoglobulin constitutes the current repertoire of immunoglobulins of said given type.

The diversity of B cell repertoire is strictly correlated with the diversity of the antibodies they express and produce. Antibody diversity is achieved in multiple ways. The rearrangement of V gene segments together with D and J segments for the heavy chain and V genes and J segments for the light chain allows combinational diversity by association of different sets of genes. The two recombinase activating genes -RAG1 and RAG2 are responsible for the V(D)J recombination process (Schatz and al. (1989). Cell 59, 1035-48; Oettinger and al. (1990). Science 248, 1517-23). Imprecise junctions of those gene segments, either by nibbling or by random addition of nucleotides by the Tdt enzyme increase again the diversity level (Bollum, F. J. (1978). Adv Enzymol Relat Areas Mol Biol 47, 347-74; Komori and al. (1993). Science 261, 1171-5; Gilfillan and al. (1993). Science 261, 1175-8). Antigen-driven affinity maturation introducing somatic hypermutations (SHM) in the V region is another step in generating diversity, as well as the process of heavy and light chain pairing (Wu and al. (2003). J Clin Immunol 23, 235-46). Class switch recombination (CSR), resulting in the production of several antibody isotypes, increases the diversity and functionality of the B cell repertoire, allowing one given variable region to be associated with different constant regions (Honjo and al. (2002) Annu Rev Immunol 20, 165-96). Finally, in some species, antibody diversification is mainly achieved by gene conversion (Thompson and Neiman (1987). Cell 48, 369-78; Reynaud and al. (1987). Cell 48, 379-88). Recently, it has been demonstrated that the three processes, SHM, CSR and gene conversion, require a single enzyme, the activation-induced cytidin desaminase (AID) (Muramatsu and al. (2000). Cell 102, 553-63; Revy and al. (2000). Cell 102, 565-75; Arakawa and al. (2002). Science 295, 1301-6; Okazaki and al. (2002). Nature 416, 340-5; Yoshikawa and al. (2002). Science 296, 2033-6). While V(D)J recombination and Tdt activity contribute to diversity generation of both T and B cell repertoires (Tuaillon and Capra (2000). J Immunol 164, 6387-97; Cabaniols and al. (2001). J Exp Med 194, 1385-90), SHM, CSR and gene conversion are B cell specific mechanisms. These additional mechanisms could eventually account for an increased diversity of the B cell repertoire as compared with that of T cells.

It represents a monumental task to describe the collective antibodies (Ab or immunoglobulins Ig) expressed at a given moment in a subject, since the immunoglobulin repertoire probably contains millions of different molecules. Only a small number of reagents capable of specifically recognizing elements of such a repertoire is as yet available. It is, of course, possible to work more finely by determining the sequence of a certain number of expressed genes. However, practical considerations make it scarcely conceivable to analyze routinely more than about ten or, perhaps, a hundred genes, and the operation is expensive and very lengthy. In short, the repertoire of immunoglobulins is described at the present time only by means of a small number of parameters.

Hence methods are not available which permit a rapid and effective analysis of the physiological and pathological situations associated with the statement of these repertoires. For example, it is clear that these repertoires vary during a voluntary immunization (vaccine), or during infection by pathogenic microorganisms, or during the progress of autoimmune pathologies. In the latter case, there are many reasons to believe that predisposition to these diseases mirrors a certain composition of the repertoires. It is hence very probable that a good method of analysis of the repertoires would have medical spin-offs, and could form the basis of techniques of medical analysis and of diagnosis.

Human T cell repertoire diversity has been analysed using different approaches (see the european patents granted under the numbers EP 566685 and EP 672184). Limiting dilutions analyses have shown that CD4 T cells from healthy individuals display a highly diverse TCR-ß repertoire (Wagner and al. (1998). Proc Natl Acad Sci U S A 95, 14447-52). Based on Immunoscope technologies, the inventors have estimated the lower limit of the total αβ TCR diversity in humans to be 25x$10^6$ different TCRs (Arstila and al. (1999). Science 286, 958-61]. Furthermore, the overall diversity of TCR-β memory CD8 T cell repertoire was estimated to be 5.$10^4$ to $10^5$ clonotypes (Arstila and al. (1999). Science 286, 958-61; Baron and al. (2003). Immunity 18, 193-204). On the contrary, little is known about the size and diversity of the peripheral blood human B cell repertoire. Most of the studies on B cell repertoire have been performed either in pathological situations or following repeated immunizations, preventing any extrapolation on the global size and diversity under-physiological conditions.

The contribution of somatic mutations to B cell repertoire diversity has been widely studied, but most studies have been carried out in murine models, determining the proportion of a mutated VH gene by a compararison with its germline sequence, as first shown by Gojobori et al. (1986, Mol Biol Evol 3, 156-67). The conclusions of those studies was that peripheral blood lymphocytes from most mice are unmutated (less than 5%) (Schittek and Rajewsky (1992). J Exp Med 176, 427-38) and that most mutated B lymphocytes are localized in germinal centers (Berek and al. (1991). Cell 67, 1121-9; MacLennan and Gray (1986). Immunol Rev 91, 61-85). A more recent study has focused on the contribution of somatic mutations to the diversity of murine serum immunoglobulins (Williams and al. (2000). Immunity 13, 409-17). Mutations do not seem to be involved in the diversity of serum IgM, IgG and IgA in young mice, but they accumulate with age in response to environmental antigens and in much higher proportions within IgG than in IgM. (Williams and al. (2000). Immunity 13, 409-17). In humans, the proportion of peripheral blood B lymphocytes bearing somatic mutations is much higher compared to mice, reaching up to 40% of all peripheral B lymphocytes, and they were shown to express the CD27 marker. (Klein and al. (1997). Blood 89, 1288-98; Klein and al. (1998). J Exp Med 188, 1679-89). However, these studies did not bring information on the global B cell clone size and on the proportion of clonal expansion within a non antigen selected rearrangement.

Similarly, two studies have been conducted for the immunoglobulins, namely Loembé and al, Eur J Immunol. 2002 Dec;32(12):3678-88, and Fais and al, J Clin Invest. 1998 Oct 15;102(8):1515-25.

The immunoscope method, based on PCR techniques, allows the quantitative study of the lymphocytes T repertoires by determining the length of the CDR3 regions and TCR, quantifying the use of the variable segments and optionally elucidating their complete sequences. The use of these methodologies for studying the human immunoglobulin repertoires and the possibility to quantify the use of the variable segments in the formation of the immunoglobulin heavy chains, although often mentioned, have never realized.

The object of the present invention is thus to provide the tools which are necessary for enabling the quantitative study of the lymphocyte B repertoire in mammals. These studies can be conducted in physiological conditions, for example within the context of the follow-up of a vaccine whose protective power comes from the production of specific antibodies, or in pathological conditions, for example when an auto-immune disease arises with the production of autoantibodies. Thus, the new application range described herein allows the follow-up of pathologies which are specifically derived from the immunoglobulin expression by the B lymphocytes and therefore distinct from that which are specifically derived from the T lymphocyte expression.

Thanks to their sensibility, the process of the present invention allows to determine the quantitative and qualitative profile of the repertory of a given type of an immunoglobulin heavy chain expressed by a lymphocyte B population, thereby determining the specific clones of lymphocyte B and the nucleic acid sequences of the genes encoding the immunoglobulins. This determination allows to follow more individually each of the cellular clones in various samplings of a given subject.

The present invention provides advantages over the prior art: firstly, the obtention of quantitative results doesn't require DNA sequencing, unlike the "unique cell PCR technique", which is much more difficult to perform and which is only available with populations previously sorted. Secondly, the results can be deduced from a large number of cells and thus are more significative in a statistical point of vue, always in comparison with the "unique cell PCR technique". Thirdly, because no antibody specific for the VH subgroups exists, the FACS quantification technique used for studying the specific lymphocyte T Vβ subgroups is not applicable to the B lymphocytes. And finally, unlike the ELISA technique which allows to describe at the proteic level the specificity and the type of the circulating antibodies, the B immunoscope technology allows to quantitatively analyse at the mRNA level the repertoire of the intracytoplasmic immunoglobulins or of the immunoglobulins expressed at the surface of the B lymphocytes, thereby providing earlier and more detailed informations of the activity state of the genes concerned.

As a consequence, the present invention provides the obtention of quantitative and qualitative informations directly from the lymphocyte B repertoire without any sequencing, it provides a rapid method, and gives the possibility to distinguish and quantify the VH repertoire specific of each immunoglobulin type individually or as a whole.

Thus, a first object of the present invention is to provide a process for determining the quantitative and qualitative profile of the repertoire of a given type of an immunoglobulin heavy chain expressed by a lymphocyte B population present in a tissue sample, characterized in that it comprises the following steps:

(a) obtaining either the cDNA from the mRNA expressed from the tissue sample or the cellular DNA extract of the tissue sample,

(b) performing the amplification of the cDNA obtained at the step (a) with a set of VH forward primers capable of specifically hybridizing in stringent conditions with the nucleic acids encoding the variable segments (VH) of immunoglobulin heavy chains, said variable segments being distributed among VH subgroups, associated with a CH reverse primer, or a mixture thereof, capable of specifically hybridizing in stringent conditions with the nucleic acid encoding the constant segment (CH) of a given type of an immunoglobulin heavy chain, and

(c) determining the quantitative and qualitative profile of the repertoire of said type of immunoglobulin heavy chain for each VH subgroup.

The terms of antibody and immunoglobulin, which are indifferently used herein, refer to the association of two heavy chains and two light chains. The part of the antibody which is specific for an antigen is constituted by the rearrangement of three gene segments V D and J for the heavy chains and V and J for the light chains. The variable segments VH of the heavy chains are classified in VH subgroups relative to the sequences of the nucleic acids which encode them. In humans, the VH subgroups are at least the VH1, VH2, VH3a, VH3b, VH4, VH5, VH6 and VH7 subgroups. Accordingly, the JH subgroups are at least the JH1, JH2, JH3, JH4, JH5 and JH6 subgroups.

The quantitative and qualitative profile of the repertoire of a given type of an immunoglobulin heavy chain refers in the present invention to the profile corresponding at once to the relative use of the nucleic acids encoding the heavy chains of immunoglobulins of a given type, in particular the nucleic acids encoding the variable segments (VH) of immunoglobulin heavy chains of a given type, expressed by a lymphocyte B population and to the length of the VDJ rearrangements for each VH or JH subgroup.

The repertoire of a given type of an immunoglobulin heavy chain refers to the immunoglobulin heavy chains of a given type which are expressed by a lymphocyte B population present in a tissue sample.

The type, or class, of an immunoglobulin heavy chain may be any type which is sufficiently described in the literature, such as the IgM type, the IgG type, in particular the IgG1 type, the IgG2 type, the IgG3 type, or the IgG4 type, the IgE type, the IgA type or the IgD type. Thus, for example, the quantitative and qualitative profile will be determined for the IgM type with the process according to the present invention.

Indeed, the lymphocytes B are subjected to the isotypic commutation: for the same variable segment, an immunoglobulin can belong to a given type and so have different properties relative to its type. The IgM are the most present immunoglobulins in the serum, the IgG correspond generally to an anamnestic reaction, the IgA are mostly expressed by the gut mucosa and the IgE are characteristic for an allergic response.

The size of a B lymphocyte clone may vary in an important manner in the course of time or after an immune response. The process for determining the quantitative and qualitative profile of the present invention allows to detect directly, without inevitably sequencing them, the presence of clonal expansions in a given rearrangement.

The tissue sample comprising the lymphocyte B population is usually the lymphocyte B population present in the blood of a subject, but it be may also tissue sample of the lymphoid system, such as for example lymph nodes or tonsil. The process for determining the quantitative and qualitative profile of the present invention doesn't necessarily require to previously purify the lymphocyte B population from the tissue sample, thus allowing to greatly facilitate its implemantation. Accordingly, the process may be performed from a heterogeneous cellular population, but also from a previously purified lymphocyte B population, or from a lymphocyte B subpopulation, such as for example naive B lymphocytes versus the memory B cells or according to the antigenic specificity antibodies they express, and therefore performing previous classical purification techniques such as magnetic sorting or any cellular sorting technique.

The cellular nucleic acid content of the tissue sample is then obtained from the tissue sample using conventional techniques. When this nucleic acid content is mRNA, the cDNA synthesis performed thanks to the reverse transcription reaction is well known, as well as the method allowing to only transcribe then mRNA present in the total RNA of the tissue sample thanks to a poly(T)primer (see Example 1: "RNA and cDNA preparation"). The nucleic acid content may also be the DNA extract of the tissue sample (see EP 566685).

Methods of DNA engineering are sufficiently described in the literature, and are well known by the man skilled in the art, who will know how to use them in the most convenient manner in the process of the invention (see Sambrook et al. (1989), Molecular Cloning: A Laboratory Manual, Cold Spring Harbor Laboratory Press, Cold Spring Harbor, N.Y.; by Ausubel et al. (1994), eds Current Protocols in Molecular Biology, Current Protocols Press; and by Berger and Kimmel (1987), Methods in Enzymology: Guide to Molecular Cloning Techniques, Vol. 152, Academic Press, Inc., San Diego, Calif.

Then the amplification is performed using a PCR type technique, that is to say the PCR technique or any other apparented technique: two primers (VH and CH); respectively complementary to the variable segment and to the constant segment of a given type of Ig heavy chain are then added to the nucleic acid content (cDNA or DNA) along with a polymerase such as Taq polymerase, and the polymerase amplifies the cDNA region between the VH and CH primers.

The expression "specifically hybridizing in stringent conditons" refers in the present invention to a hybridizing step in the process of the invention where the oligonucleotide sequences selected as probes or primers are of adequate length and sufficiently unambiguous so as to minimize the amount of non-specific binding that may occur during the amplification.

Hybridization is typically accomplished by annealing the oligonucleotide probe or primer to the cDNA (or DNA) under conditions of stringency that prevent non-specific binding but permit binding of this cDNA which has a significant level of homology with the probe or primer.

Among the conditions of stringency is the melting temperature Tm for the amplification step using either the set of VH forward primers associated with the CH primer, or the VH internal forward primer associated with the set of JH reverse primers, which is in the range of about 58°C to about 60°C ; preferably, the Tm for the amplification step is in the range of about 58°C or about 60°C. Most preferably, the Tm for the amplification step is about 60°C.

Typical hybridization and washing stringency conditions depend in part on the size (i.e., number of nucleotides in length) of the cDNA or oligonucleotide probe (Ausubel and al., 1994, eds Current Protocols in Molecular Biology).

The oligonucleotide probes or primers herein described may be prepared by any suitable methods such as chemical synthesis methods (see references supra for methods of DNA engineering).

Preferably, the process for determining the quantitative and qualitative profile according to the present invention is characterized in that separated amplifications are performed for each of the VH subgroups.

Indeed, the nucleic acid content (cDNA or DNA) obtained at the step (a) from the tissue sample is divided such as to perform so much separated amplifications as there are couples of VH and CH primers.

By the expression mixture of CH reverse primer it is intended to refer in the present invention to a mixture of at least two, preferably at least 2, 3, 4 or at least 5 CH reverse primers, said CH reverse primers being preferably present in an equivalent quantity between each other. For example, when there are 2 CH reverse primers in the mixture, each CH reverse primer is present at 50 % in said mixture.

In a preferred embodiment, the process for determining the quantitative and qualitative profile according to the present invention is characterized in that the separated amplifications are real-time separated amplifications, said real-time amplifications being performed using a CH labeled reverse probe, prferably a CH labeled reverse hydrolysis-probe, capable of specifically hybridizing in stringent conditions with the constant segment of the given type of immunoglobulin heavy chain and capable of emiting a detectable signal everytime each amplification cycle occurs, and characterized in that the signal obtained for each VH subgroup is measured.

The real-time amplification, such as real-time PCR, is well known by the man skilled in the art, and the various known techniques will be employed in the best way for the implementation of the present process. These techniques are performed using various categories of probes, such as hydrolysis probes (TaqMan, Applied Biosystems, USA), hybridization adjacent probes, or molecular beacons. Hydrolysis probes are preferred. The techniques employing hydrolysis probes or molecular beacons are based on the use of a fluorescence quencher/reporter system, and the hybridization adjacent probes are based on the use of fluorescence acceptor/donor molecules.

Hydrolysis probes with a fluorescence quencher/reporter system are available in the market, and are for example commercialized by the Applied Biosytems group (USA). Many fluorescent dyes may be employed, such as FAM dyes (6-carboxy-fluorescein), or any other dye phosphoramidite reagents. In the present invention, the inventors have employed a TaqMan Minor Groove Binder (MGB) FAM-labeled probe (see Example 1), but other probes may be used, in a convenient manner.

Among the stringent conditions applied for anyone of the hydrolysis-probes of the present invention is the Tm, which is in the range of about 68°C to 70°C ; preferably, the Tm for anyone of the hydrolysis-probes of the present invention is in the range of about 69°C to about 70°C. Most preferably, the Tm applied for anyone of the hydrolysis-probes of the present invention is about 70°C.

In another preferred embodiment, the process for determining the quantitative and qualitative profile according to the present invention is characterized in that the separated amplification products obtained for each of the VH subgroups are further elongated using a CH labeled reverse probe capable of specifically hybridizing in stringent conditions with the constant segment of the given type of immunoglobulin heavy chain and capable of emiting a detectable signal, and characterized in that the elongation products are separated, for each of the VH subgroups, relative to their length, the signal obtained for the separated elongation products is measured, and the quantitative and qualitative profile of the labeling intensity relative to the elongation product length is established, for each of the VH subgroups individually.

This elongation step, also named "run-off reaction", allows to determine the length of the VDJ rearrangements fro each VH subgroup. The separated amplification products obtained for each VH subgroup are elongated using a DNA polymerase and a CH labeled reverse probe capable of specifically hybridizing in stringent conditions with the constant segment of the given type of immunoglobulin heavy chain and capable of emiting a detectable signal. Elongation products are thus obtained, which are labeled at their CH end and their length can be determined.

The determination of the length can be realized using conventional techniques for the determination of DNA sequences, with gels such as polyacrylamide gels for the separation, DNA sequencers and adapted softwares. The labeling of the CH labeled reverse probe may be any appropriate labeling, such as for example radio-labeling or preferably fluorescent-labeling. The "run-off reaction" is well described in EP 566 685.

Among the stringent conditions applied for anyone of the labeled probes used for the elongation ("run-off reaction) is the Tm, which is in the range of about 58°C to about 60°C ; preferably, the Tm for the amplification step is in the range of about 58°C or about 60°C. Most preferably, the Tm for the amplification step is about 60°C.

Preferably, the process for determining the quantitative and qualitative profile according to the present invention is characterized in that the set of VH forward primers comprises at least the 8 following subgroups of VH primers corresponding to the VH subgroups :

- the VH 1 primers having the sequences SEQ ID N° 1 to SEQ ID N° 3, and
- the VH2 primer having the sequence SEQ ID N° 4, and
- the VH3a primers having the sequences SEQ ID N° 5 and SEQ ID N° 6, and
- the VH3b primers having the sequences SEQ ID N° 7 to SEQ ID N° 10, and
- the VH4 primers having the sequences SEQ ID N° 11 and SEQ ID N° 12, and
- the VH5 primer having the sequence SEQ ID N° 13, and
- the VH6 primer having the sequence SEQ ID N° 14, and
- the VH7 primer having the sequence SEQ ID N° 15.

More preferably, the process for determining the quantitative and qualitative profile according to the present invention is characterized in that the sequences SEQ ID N° 1 to SEQ ID N° 15 may contain at least one to three point mutations, except for the nucleotides 1 to 6 of their 3' part.

The point mutation refers in the present invention to a mutation which occurs for only one nucleotide, on the contrary of a mutations which occurs for an oligonucleotide sequence. The point mutation may be a substitution, a deletion or an addition.

The process for determining the quantitative and qualitative profile according to the present invention may further be characterized in that the CH reverse primer is selected from the CH reverse primers capable of specifically hybridizing in stringent conditions with the nucleic acids encoding the constant segments (CH) of the IgM heavy chain, the IgE heavy chain and the IgA heavy chain.

Preferably, the process for determining the quantitative and qualitative profile according to the present invention is characterized in that, when the CH reverse primer is capable of specifically hybridizing in stringent conditions with the nucleic acid encoding the constant segment (CH) of the IgM heavy chain, the CH reverse primer has the sequence SEQ ID N° 26, or the sequence SEQ ID N° 26 wherein one to three point mutations may occur, except for the nucleotides 1 to 6 of its 3' part.

In another preferred embodiment, the process for determining the quantitative and qualitative profile according to the present invention is characterized in that, when the CH reverse primer is capable of specifically hybridizing in stringent conditions with the nucleic acid encoding the constant segment (CH) of the IgE heavy chain, the CH reverse primer has the sequence SEQ ID N° 33, or the sequence SEQ ID N° 33 wherein one to three point mutations may occur, except for the nucleotides 1 to 6 of its 3' part.

In another preferred embodiment, the process for determining the quantitative and qualitative profile according to the present invention is characterized in that, when the given type of immunoglobulin heavy chain is the IgG type, a mixture of two CH reverse primers is associated with the set of VH forward primers, said two CH reverse primers having the sequences SEQ ID N° 27 and SEQ ID N° 28, or the sequences SEQ ID N° 27 and SEQ ID N° 28 wherein one to three point mutations may occur, except for the nucleotides 1 to 6 of its 3' part.

Preferably, the process for determining the quantitative and qualitative profile according to the present invention may further be characterized in that the CH reverse primer is selected from the CH reverse primers capable of specifically hybridizing in stringent conditions with the nucleic acids encoding the constant segments (CH) of the IgG1 heavy chain, the IgG2 heavy chain, the IgG3 heavy chain or the IgG4 heavy chain.

Furthermore, the process for determining the quantitative and qualitative profile according to the present invention is characterized in that, when the given type of immunoglobulin heavy chain is an IgM heavy chain and when the separated amplifications are real-time separated amplifications, the CH labeled hydrolysis-probe has the sequence SEQ ID N° 29, or the sequence SEQ ID N° 29 wherein at least one point mutation may occur.

Furthermore, the process for determining the quantitative and qualitative profile according to the present invention is characterized in that, when the given type of immunoglobulin heavy chain is an IgM heavy chain and when the separated amplification products obtained for each of the VH subgroups are further elongated, the CH labeled reverse probe has the sequence SEQ ID N° 30, or the sequence SEQ ID N° 30 wherein one to three point mutations may occur, except for the nucleotides 1 to 6 of its 3' part.

Preferably, the process for determining the quantitative and qualitative profile according to the present invention is characterized in that, when the given type of immunoglobulin heavy chain is an IgE heavy chain and when the separated amplifications are real-time separated amplifications, the CH labeled hydrolysis-probe has the sequence SEQ ID N° 36, or the sequence SEQ ID N° 36 wherein at least one point mutation may occur.

Furthermore, the process for determining the quantitative and qualitative profile according to the present invention is characterized in that, when the given type of immunoglobulin heavy chain is an IgE heavy chain and when the separated amplification products obtained for each of the VH subgroups are further elongated, the CH labeled reverse probe has the sequence SEQ ID N° 37, or the sequence SEQ ID N° 37 wherein one to three point mutations may occur, except for the nucleotides 1 to 6 of its 3' part.

Preferably, the process for determining the quantitative and qualitative profile according to the present invention is characterized in that, when the given type of immunoglobulin heavy chain is an IgG heavy chain and when the separated amplifications are real-time separated amplifications, the CH labeled hydrolysis-probe has the sequence SEQ ID N° 34, or the sequence SEQ ID N° 34 wherein at least one point mutation may occur.

Furthermore, the process for determining the quantitative and qualitative profile according to the present invention is characterized in that, when the given type of immunoglobulin heavy chain is an IgG heavy chain and when the separated amplification products obtained for each of the VH subgroups are further elongated, the CH labeled reverse probe has the sequence SEQ ID N° 35, or the sequence SEQ ID N° 35 wherein one to three point mutations may occur, except for the nucleotides 1 to 6 of its 3' part.

Preferably, the CH labeled hydrolysis-probes having the sequences SEQ ID N° 29, SEQ ID N° 34 and SEQ ID N° 36, may have 2 point mutations in their sequences.

The point mutations which may occur in the sequences of these CH labeled hydrolysis-probes may be any point mutations provided that this sequence is of adequate length and sufficiently unambiguous so as to minimize the amount of non-specific binding that may occur.

In one further embodiment, the process for determining the quantitative and qualitative profile according to the present invention is characterized in that further separated amplifications for each of JH subgroups are performed from the separated amplification products obtained for at least one given VH subgroup of the VH subgroups with the CH reverse primer, said further separated amplifications being performed using a VH internal forward primer corresponding to the given VH subgroup, and associated with a set of JH reverse primers corresponding to the JH subgroups and capable of specifically hybridizing in stringent conditions with the nucleic acids encoding the junction segments of the given type of immunoglobulin heavy chain.

The stringent conditions applied for the further separated amplifications using the VH internal forward primer associated with the set of JH reverse primers are the same as that applied for the amplification step using the set of VH forward primers associated with the CH primer (see supra). Most preferably, the Tm for the amplification step using the VH internal forward primer associated with the set of JH reverse primers is about 60°C.

Preferably, the process for determining the quantitative and qualitative profile according to the present invention is characterized in that the further separated amplifications are real-time amplifications performed using a VH labeled forward probe, preferably a VH labeled forward hydrolysis-probe, capable of specifically hybridizing in stringent conditions with the variable segment of the given type of immunoglobulin heavy chain and capable of emiting a detectable signal everytime each amplification cycle occurs, and characterized in that the signal obtained for each JH subgroup is measured.

Among the stringent conditions applied for the VH labeled forward hydrolysis-probe capable of specifically hybridizing with the variable segment of the given type of immunoglobulin heavy chain is the Tm, which is in the range of about 68°C to about 70°C ; preferably, the Tm for the VH labeled forward hydrolysis-probe is in the range of about 69°C to about 70°C ; Most preferably, the Tm applied for the VH labeled forward hydrolysis-probe-is about 70°C.

More preferably, the process for determining the quantitative and qualitative profile according to the present invention is characterized in that, when the given VH subgroup is the VH5 subgroup, the VH5 internal forward primer has the sequence SEQ ID N° 31, or the sequence SEQ ID N° 31 wherein one to three point mutations may occur, except for the nucleotides 1 to 6 of its 3' part.

More preferably, the process for determining the quantitative and qualitative profile according to the present invention is characterized in that the VH labeled forward hydrolysis-probe has the sequence SEQ ID N° 32, or the sequence SEQ ID N° 32 wherein at least one point mutation may occur.

Preferably, the VH labeled forward hydrolysis-probe having the sequence SEQ ID N° 32 may have 2 point mutations in its sequence.

The point mutations which may occur in the sequences of this VH labeled forward hydrolysis-probe may be any point mutations provided that this sequence is of adequate length and sufficiently unambiguous so as to minimize the amount of non-specific binding that may occur.

In one further embodiment, the process for determining the quantitative and qualitative profile according to the present invention is characterized in that separated elongations are performed for each of the JH subgroups from the separated amplification products obtained for at least one given VH subgroup of the VH subgroups with the CH reverse primer,

said further separated elongations being performed using a set of JH labeled reverse primers corresponding to JH subgroups and capable of specifically hybridizing in stringent conditions with the nucleic acids encoding the junction segments of the given type of immunoglobulin heavy chain, said JH labeled reverse primers being capable of emiting a detectable signal,
and characterized in that the elongation products are separated, for each of the JH subgroups, relative to their length, the signal obtained for the separated elongation products is measured, and the quantitative and qualitative profile of the labeling intensity relative to the elongation product length is established, for each of the JH subgroups for the given VH subgroup.

The stringent conditions applied for the set of JH labeled reverse primers used for the elongation and capable of specifically hybridizing with the junction segment of the given type of immunoglobulin heavy chain is the Tm, which is in the range of about 58 and about 60 °C, preferably, about 59 °C, most preferably, about 60 °C.

Preferably, the process for determining the quantitative and qualitative profile according to the present invention is characterized in that the set of JH forward primers, optionally labeled, comprises at least the 6 following subgroups of JH primers corresponding to the JH subgroups :

- the JH1 primer having the sequence SEQ ID N° 16, and
- the JH2 primer having the sequence SEQ ID N° 17, and
- the JH3 primer having the sequence SEQ ID N° 18, and
- the JH4 primers having the sequences SEQ ID N° 19 to SEQ ID N° 21, and
- the JH5 primer having the sequence SEQ ID N° 22, and
- the JH6 primers having the sequences SEQ ID N° 23 to SEQ ID N° 25.

More preferably, the process for determining the quantitative and qualitative profile according to the present invention is characterized in that the sequences SEQ ID N° 16 to SEQ ID N° 25 may contain at least one to three point mutations, except for the nucleotides 1 to 6 of their 3' part.

One another subject of the present invention is a set of VH forward primers capable of specifically hybridizing in stringent conditions with the nucleic acids encoding the variable segments (VH) of immunoglobulin heavy chains, said variable segments being distributed among at least 8 VH subgroups, associated with a CH reverse primer, or a mixture thereof, capable of specifically hybridizing in stringent conditions with the nucleic acid encoding the constant segment (CH) of a given type of an immunoglobulin heavy chain, characterized in that the set of VH forward primers comprises at least the 8 following subgroups of VH primers corresponding to the VH subgroups :

- the VH1 primers having the sequences SEQ ID N° 1 to SEQ ID N° 3, and
- the VH2 primer having the sequence SEQ ID N° 4, and
- the VH3a primers having the sequences SEQ ID N° 5 and SEQ ID N° 6, and
- the VH3b primers having the sequences SEQ ID N° 7 to SEQ ID N° 10, and
- the VH4 primers having the sequences SEQ ID N° 11 and SEQ ID N° 12, and
- the VH5 primer having the sequence SEQ ID N° 13, and
- the VH6 primer having the sequence SEQ ID N° 14, and
- the VH7 primer having the sequence SEQ ID N° 15.

Preferably, the set of VH forward primers according to the present invention is characterized in that the sequences SEQ ID N° 1 to SEQ ID N° 15 may contain at least one to three point mutations, except for the nucleotides 1 to 6 of their 3' part.

More preferably, the set of VH forward primers according to the present invention is characterized in that the CH reverse primer is selected from the CH reverse primers capable of specifically hybridizing in stringent conditions with the nucleic acids encoding the constant segments (CH) of the IgM heavy chain, the IgE heavy chain and the IgA heavy chain.

Preferably, the set of VH forward primers according to the present invention is characterized in that, when the CH reverse primer is capable of specifically hybridizing in stringent conditions with the nucleic acid encoding the constant segment (CH) of the IgM heavy chain, the CH reverse primer has the sequence SEQ ID N° 26, or the sequence SEQ ID N° 26 wherein one to three point mutations may occur, except for the nucleotides 1 to 6 of its 3' part.

In another preferred embodiment, the set of VH forward primers according to the present invention is characterized in that, when the CH reverse primer is capable of specifically hybridizing in stringent conditions with the nucleic acid encoding the constant segment (CH) of the IgE heavy chain, the CH reverse primer has the sequence SEQ ID N° 33, or the sequence SEQ ID N° 33 wherein one to three point mutations may occur, except for the nucleotides 1 to 6 of its 3' part.

In another preferred embodiment, the set of VH forward primers according to the present invention is characterized in that, when the given type of immunoglobulin heavy chain is the IgG type, a mixture of two CH reverse primers is associated with the set of VH forward primers, said two CH reverse primers having the sequences SEQ ID N° 27 and SEQ ID N° 28, or the sequences SEQ ID N° 27 and SEQ ID N° 28 wherein one to three point mutations may occur, except for the nucleotides 1 to 6 of its 3' part.

Preferably, the set of VH forward primers according to the present invention according to the present invention may further be characterized in that the CH reverse primer is selected from the CH reverse primers capable of specifically hybridizing in stringent conditions with the nucleic acids encoding the constant segments (CH) of the IgG1 heavy chain, the IgG2 heavy chain, the IgG3 heavy chain or the IgG4 heavy chain.

Another subject of the present invention is a method for the *in vitro* diagnosis of a condition associated with an abnormal expression of the repertoire of a given type of an immunoglobulin heavy chain by a lymphocyte B population in a subject, characterized in that it comprises the-following steps:

(1) determining the quantitative and qualitative profile of the given type of immunoglobulin heavy chain from a tissue sample of said subject according to the present invention,
(2) comparing the quantitative and qualitative profile obtained at the step (1) with a control quantitative and qualitative profile of said given type of immunoglobulin heavy chain, the demonstration of a significant modification of the profile obtained at the step (1) being significant of such a condition in the subject.

Preferably, the method for *the in vitro* diagnosis according to the present invention is characterized in that the condition is an auto-immune disease, a B cell lymphoma or an immunodepressive disease.

In another preferred embodiment, the method for *the in vitro* diagnosis according to the present invention is characterized in that the condition results from a bone marrow transplantation, from a vaccinal test or from an allergic reaction.

Another subject of the present invention is a method for the *in vitro* follow-up of a treatment of a condition associated with an abnormal expression of the repertoire of a given type of an immunoglobulin heavy chain by a lymphocyte B population in a subject, characterized in that it comprises the following steps:

(1) optionally, determining before the treatment the quantitative and qualitative profile of the given type of immunoglobulin heavy chain from a tissue sample of said subject according to the present invention,
(2) determining, during the treatment, the quantitative and qualitative profile of the given type of immunoglobulin heavy chain at given times from tissue samples of said subject according to the present invention,
(3) comparing the quantitative and qualitative profiles obtained at the step (2) and optionally at the step (1) with each others and optionally with a control quantitative and qualitative profile of the given type of immunoglobulin heavy chain, the demonstration of a significant modification of the profile obtained at the step (1) being significant of such a condition in the subject.

Preferably, the method for the *in vitro* follow-up according to the present invention is characterized in that the condition is an auto-immune disease, a B cell lymphoma or an immunodepressive disease.

In another preferred embodiment, the method for the *in vitro* follow-up according to the present invention is characterized in that the condition results from a bone marrow transplantation, from a vaccinal test or from an allergic reaction.

Another subject of the present invention is a kit for determining the quantitative and qualitative profile of the repertoire a given type of an immunoglobulin heavy chain expressed by a lymphocyte B population present in a tissue sample, characterized in that it comprises the set of VH forward primers according to the present invention associated with the CH reverse primer.

Preferably, the kit according to the present invention is characterized in that it further comprises a set of JH reverse primers, optionally labeled, corresponding to the JH subgroups and capable of specifically hybridizing in stringent conditions with the nucleic acids encoding the junction segments of the given type of immunoglobulin heavy chain.

More preferably, the kit according to the present invention is characterized in that the set of JH reverse primers comprises the 6 following subgroups of JH primers corresponding to the JH subgroups :

- the JH1 primer having the sequence SEQ ID N° 16, and
- the JH2 primer having the sequence SEQ ID N° 17, and
- the JH3 primer having the sequence SEQ ID N° 18, and
- the JH4 primers having the sequences SEQ ID N° 19 to SEQ ID N° 21, and
- the JH5 primer having the sequence SEQ ID N° 22, and
- the JH6 primers having the sequences SEQ ID N° 23 to SEQ ID N° 25.

More preferably, the kit according to the present invention is characterized in that the sequences SEQ ID N° 16 to SEQ ID N° 25 may contain at least one to three point mutations, except for the nucleotides 1 to 6 of their 3' part.

Kits comprising primers according to the present invention are well described in the literature and may further comprise suitable reagents.

Another subject of the present invention is the use of the kit according to the present invention, for the *in vitro* diagnosis of a condition associated with an abnormal expression of the repertoire of a given type of an immunoglobulin heavy chain by a lymphocyte B population in a subject.

Preferably, the use of the kit according to the present invention is characterized in that the condition is an auto-immune disease, a B cell lymphoma or an immunodepressive disease.

In another preferred embodiment, the use of the kit according to the present invention is characterized in that the condition results from a bone marrow transplantation, from a vaccinal test or from an allergic reaction.

The purpose of the legends of the figures and of the examples below is to illustrate the invention. It doesn't limit the scope of the claimed invention.

## LEGENDS OF THE FIGURES

### Figure 1: Immunoscopes profiles from two healthy donors.

In Figure 1A, purified B cells from two healthy donors, donors 789 and 743 were subjected to VH families specific PCR amplification as detailed in Materiel and Methods using VH specific primers (see Table 2) and Cμ primer, followed by a "run-off" reaction with a Cμ-Fam probe. For donor 743, Immunoscope profiles were obtained from two separate samples, sample W and Z, harboring the same initial number of B cells. In Figure 1B, Immunoscope profiles were realized for two given rearrangements, VH5-JH1 and VH5-JH2. For both 789 and 743 donors, VH5-JH1 rearrangement have been chosen as prototype rearrangement for diversity determination, and subjected to exhaustive sequencing. In addition, purified CDR3 band from VH5-JH2 rearrangement of donor 789 (shown by arrow) was also used for the same purpose. This purified band used have a CDR3 length of 8 aminoacids.

### Figure 2: Two different examples of IgM clonal expansions

Clonal expansion A has been found in the course of VH5-JH2 exhaustive sequencing from donor 749. All the sequences from clonal expansion A have the same CDR3 of 14 aminoacids with the sequence SEQ ID N°38. Clonal expansion B have been found in the course of VH5-JH1 exhaustive sequencing from donor 749. All the sequences from clonal expansion B have the same CDR3 of 25 aminoacids with the sequence SEQ ID N°39. The sequences differs in the mutations occuring in the VH5 region. Codons where mutations occur are initiated in red according to standart nomenclature.* or ** means differents mutations occuring on the same codon. Numbers of each clone for a given pattern of mutations are indicated in black. All mutations occurs from the germline clones (GL). Dashed clones are not found virtual intermediate clones. All existing clones are denominated by a letter.

### Figure 3: Properties and distribution of mutated versus germlime sequences

VH5-JH1 exhaustive sequencing from donor 743 was realized separately in two samples, W and Z containing the same number of B cells. (See Table 4). Figure 3A represent the distribution of the sequences between the two samples. tW, tZ and tOv stand for total sequences of W, Z and W-Z Overlap respectively, dW, dZ and dOv means the different sequences in W, Z and W-Z Overlap respectively. Figure 3B shows the distribution of germline versus mutated sequences in relation with the number of sequences found for each individual clone.

## EXAMPLES

### Example 1: Experimental procedures

### 1. Cell preparations, antibodies and flow cytometry.

Blood (approximatively 500ml) from three healthy donors was obtained from the Etablissement Français du Sang, Necker Enfants Malades Lecourbe, Paris, France. Donor 789 is a 56 year old woman, donor 743 is a 55 year old woman, donor 522 is a 30 year old man. PBMCs were isolated by centrifugation over Ficoll-Paque (Amcrsham Biosciences AB, Uppsalla, Sweden) in UN1-SEP$_{maxi+}$ tubes (Novamed, Jerusalem, Israel).

B cells from donor 789 were then obtained from the PBMCs by depletion using the B cell negative isolation kit from Dynal (Dynal, Oslo , Norway). For donors 743 and 522, PBMCs were co-stained with CD19 beads and CD19-PE (Pharmingen, San Jose, CA) and purified by positive selection on AutoMACS using respectively Possel or Possel-S programs (Miltenyi Biotec, Bergisch-Gladbach, Germany). The eluted cells from the positive and negative fractions, as well as the total PBMCs were then labeled with either anti-IgM, anti-IgG, anti-IgD, Ig-E, anti-IgA1/IgA2, anti-kappa and anti-lambda antibodies (Pharmingen, San Jose, CA) and rabbit polyclonal anti-Human IgM (Jackson Immunoresearch laboratories, West Grove, Pennsylvania).
Using these different antibodies, the percentage and the absolute number of cells bearing the different antibody isotypes were determined among the PBMC and B cells positive fraction of donor 743 and 522 (Table 1). The purity of the positive fraction was checked either by the CD 19 staining, either by the sum of anti-kappa and anti-lambda staining.

**2. RNA and cDNA preparation**

Cells from both positive and negative fractions were aliquoted and kept frozen at -20° in the lysis buffer of the RNeasy mini-kit (Qiagen, Courtaboeuf, France). Total RNA was extracted using the RNeasy mini-kit (Qiagen, Courtaboeuf, France) according to the manufacturers specifications, as previoulsly described (Lim and al. (2002). J Immunol Methods 261, 177-94). cDNA was then prepared by RNA reverse-transcription with 0,5μg/μl oligo (dT)17 and 200 U of Superscript II reverse transcriptase (Invitrogen, Cergy Pontoise, France).

**3. Quantitative Immunoscope**

Quantitative Immunoscope analysis was performed as described (Lim and al. (2002). The different VH germline genes can be clustered in seven families according to their level of homology. Both IMGT (http://imgt.cines.fr) (Lefranc and al. (1999). Nucleic Acids Res 27, 209-12) and Vbase databases (http://www.mrc-cpe.cam.ac.uk) (Tomlinson and al. (1998). V Base Sequence Directory. In, M. C. f. P. Engineering, ed. (Cambridge, UK.)) were used to have access to their sequences and the germinal gene were aligned using GCG Wisconsin package program (http://www.accelrys.com/products/gcg wisconsin_package).
Lefranc, M.P. et al., Nucleic Acids Research, vol. 29, number 1, pages 207 - 209, discloses the IMGT database, which is a high quality integrated information system specialising in immunoglobulins, T-cell receptors and major histocompatibility complex molecules. It provides a common access to all nucleotide, protein, genetic and structural immunogenetics data. However, although this publication discloses the IMGT database which may contain VH germline gene sequences, it does not specify the specific set of VH forward primers as comprised within the underlying set of claims.
All the primers used are shown in Table 2. In order to have the opportunity to study somatic mutations in the VH regions, VH family specific primers were chosen in the FR1 region for all the VH families except VH3 and VH4 families where the specific primers were designed in the FR3 region. VH3 gene family, the largest one, was divided in two sub-families VH3a and VH3b in order to achieve a better specifity. This specifity was tested by specificaly amplify each VH family from a PBMC mix of three heathly donors using VH family specific primers and HIGCM primer, PCR products cloning and sequencing. For only one VH family, the VH7 family, full specifity of the amplification was not achieved as few of the amplified VH were belonging to the VH1 family.

For the quantification of the VH families usage for IgM expressing cells, an aliquot of the cDNA was amplified using pfu high fidelity Taq polymerase (Stratagene, Amsterdam, The Netherlands) with each of the 8 families VH specific primers on one side, the HIGCM primer on the other side and the TM-MGB-HCM probe, a Taqman Minor Groove Binder (MGB) FAM-labeled nested probe specific for the Cμ region.
For the quantification of the JH usage, an aliquot of the cDNA was amplified on the 5' side with the VH5int specific primer (SEQ ID N° 31) and each of the 6 JH specific primers on the 3' side together with the TM-MGB-VH5int probe (SEQ ID N° 32), a Taqman MGB FAM-labeled nested probe specific for the VH5 family. All Taqman MGB probes were designed using the Primer express software program (Applied Biosystems, Courtaboeuf, France). PCR reactions were carried out in 25μl total volume. For all these differents reactions, real time quantitative PCR was then performed on an ABI 5700 system (Applied Biosystems, Courtaboeuf, France). The relative usage of each VH family or each JH segment was calculated according to the above formula:

$$U(IgVH_y) = \sum_{x=1}^{x=8} 2^{(C_1(x)-C_1(y))} \qquad U(IgJH_y) = \sum_{x=1}^{x=6} 2^{(C_1(x)-C_1(y))}$$

In which $C_1(x)$ is the fluorescent threshold cycle number mesured for VH(y) family or JH(y). In this case, the VH family primers pair display a mean efficiency of 0.95 ± 0.08 and the JH segment primers display a mean efficiency of 0.91 ±0.08.
For the VH family amplifications, 2μl of each amplification reactions were used as template in a run-off reaction initiated by either the HCM-FAM primer, a fluorescent nested Cμ primer, the JH1-FAM: 5'-Fam-CCCTGGCCCCAGTGCTG-3' (SEQ ID N° 16) or JH2-FAM 5'Fam-CCACGGCCCCAGAGATCG-3' (SEQ ID N° 17) primers or the VH5-FAM primer in a total volume of 10μl as previously described (Pannetier and al. (1997). In The Antigen T Cell receptor: Selected Protocols and Applications., J. R. E. Oksenberg, ed. (Landes Bioscience, Chapman & Hall), pp. 287). All fluorescent fragments were then separated on a denaturing 6% acrylamide gel, run on an automated 373 DNA sequencer (Applied Biosystems, Courtaboeuf, France) and analyzed with Immunoscope software (Pannetier and al. (1993). Proc Natl Acad Sci U S A 90, 4319-23).

### 4. CDR3 band purification

In the case of VH5-JH2 rearrangement amplification, bands corresponding to a CDR3 length of 8 amino-acids had to be purified from the acrylamide gel. For this purpose, the PCR products were made visible on the acrylamide gel by a DNA silver staining system as previously described (Promega, Madison, Wi) (Casrouge and al. (2000). J Immunol 164, 5782-7; Raaphorst and al. (1996). Biotechniques 20, 78-82, 84, 86-7). The bands of interest were cutted from the gel and disrupted in 50μl of TE. A second 33 cycles PCR using the VH5int and the JH2 primers was then conducted with 1μl of the band purification recovered PCR product and the amplification was then cloned as follow.

### 5. VH transcript sequencing and analysis

Either the total product of the VH5-JH1 rearrangement from different donors or the two PCR bands purified products amplified from the VH5-JH2 (8aa) CDR3 bands) were cloned using the TOPO Blunt PCR cloning kit (Invitrogen, Cergy Pontoise, France). Sequencing reactions were performed as previously described (Arstila and al. (1999). Science 286, 958-61) directly on these products using VH5int primer and the ABI PRISM Big Dye Terminator Cycle Sequencing Ready Reaction Kit (Applied Biosystems, Courtaboeuf, France). These sequencing reactions were then run on an ABI PRISM 3700 DNA analyser (Applied Biosystems, Courtaboeuf, France). CDR3 regions and VH mutations of the corresponding sequences were extracted and analysed using Taps1.1 software written by Emmanuel Beaudoing (Casrouge and al. (2000). J Immunol 164, 5782-7).

### 6. Calculation of the VH/Bcells repertoire size

The size of the VH repertoire can be estimated to equal the number of distinct sequences found for a given rearrangement (when sequencing is undergone to saturation) divided by the product of the considered VH frequency with the considered JH frequency. If the size of the repertoire was deduced from a purified CDR3 band, the percentage of this band among the all rearangement was taken in account in the above calculations. Diversity due to CDR3 variability can be distinguished from the diversity due to somatic mutations according to the way the sequences are analysed on the Taps software. The calculations can be done either with the number of distincts sequences given by limits flanking the CDR3 regions, or including most of the VH region.

### Example 2: Quantification of the VH and JH gene segment usage in human PBMCs

In order to estimate the size of the peripheral human B cell repertoire, we have adapted the Immmunoscope method developped in our laboratory and previously applied for T cell repertoire studies (Pannetier and al. (1993). Proc Natl Acad Sci U S A 90, 4319-23). In addition, a precise quantification of VH and JH gene usage was performed by coupling real time PCR with Immunoscope analyses. B cells from several donors were subjected to those studies. Table 1 shows representative Facs analysis of two donors, before and after B cells enrishment. cDNA prepared from those cells of two donors were then submited to a step of PCR amplification using VH family specific primers on one side and one Cμ primer, specific for the constant region of IgM on the other side, together with the TM-MGB-HCM fluorescent probe, nested in the constant region close to the Cμ primer (see Material & Methods and Table 2). Real time determination of the level of fluorescence at each PCR step makes possible the quantification of VH family usage as shown in Table 3a. For a given donor, very large difference of VH family usage could be evidenced, VH3 and VH4 gene families representing the majority of all rearrangements (60% and 20% respectively). This is compatible with a utilisation of VH gene proportional to the complexity of each family, since 27 different genes belong to the VH3 gene family and 10 to the VH4 family. Those results are also in accordance with what have been already determined by single cell PCR (54% of VH3 rearrangement and 23% of VH4 rearrangement out of 491 analysed cells) (Odendahl and al. (2000). J Immunol 165, 5970-9) althought they are derived from a much larger cell number (about 70 000 cells). In order to evaluate the reproducibility of the quantification, purified IgM positive B cells from donor 743 were divided into 6 samples (2.7 $10^6$ IgM positive cells/sample, see Table 3A). Two of these samples, sample W and sample Z were then tested for VH family usage quantification. As can be seen from Table 3a, VH usage of samples W and Z are almost identical, as expected. In addition, the values obtained for both samples are very similar to those obtained for donor 789, although individual variations could be detected, for example in the VH2 family gene usage.

The inventors then quantified of the JH usage for one given VH family. VH5 family was chosen for two main reasons. 1°) according to IMGT, the number of individual genes is limited to only two germline encoded genes, VH5 and VH5.51. 2°) data on VH gene family usage (Table3A) have shown that VH5 is not overused (1% of all rearrangement in sample W and Z from donor 743 and 3.2% for donor 789). For the quantification of JH gene utilisation, VH5-Cμ amplification PCR products from the two different donors were subjected to a second PCR amplification using VH5int primer specific for the VH5 genes on one side and each of the 6 JH specific primers on the other side, together with the TM-MGB-VH5int fluorescent probe, nested in the vicinity of the VH5int primer and specific for the VH5 genes (see Material & Methods and Table 2). As shown in Table 3B, overutilisation of JH4 was observed for both donors. Again, separate quantification of samples W and Z from donor 743 gave almost identical results. Nevertheless, overall JH usage was less similar when donors 789 and 743 were compared (Table 3B). For the rest of thes study, the inventors have focused two gene segments, JH1 and JH2, because they are the less used in all rearrangements of both donors 789 and 743.

**Example 3: Immunoscope profiles of human peripheral blood IgM positive B lymphocytes**

Following quantification of VH and JH usage, PCR amplification was submitted to a « run-off » reaction, as previously described (Pannetier and al. (1993). Proc Natl Acad Sci U S A 90, 4319-23]. Figure1A displays for each VH family the different immunoscope profils obtained from both 789 and 743 donors. Compared to T cells [Arstila and al. (1999). Science 286, 958-61], B cell profiles have two mains characteristics : 1°) the number of peaks representing a given size of CDR3 is larger, 2°) the mean size of these peaks is close to 15 amino-acids CDR3 while, for T cells, it is close to 10 amino-acids CDR3. Most patterns display a Gaussian repartition of the CDR3 length, in agrement with previously reported profiles of mouse splenic B cells [Delassus and al. (1995). J Immunol Methods 184, 219-29] although some pertubations can be observed for some of the smallest VH families (VH2 and VH6 families for donor 789).
Figure 1B shows Immunoscope profiles specific for the VH5-JH2 rearrangement in donor 789 and VH5-JH1 rearrangement in the two donors 789 and 743. The Immunoscope software allow to calculate the aera beneath each peak which is directly proportional to the number of sequences included in the peak [Pannetier and al. (1993). Proc Natl Acad Sci U S A 90, 4319-23]. Thus, for the VH5-JH2 rearrangement in donor 789, the peak corresponding to a CDR3 length of 8 aminoacids represents 1.84% of all the VH5-JH2 rearrangement. Similarely the peaks corresponding to different CDR3 length of the VH5-JH2 rearrangement for the donor 789 (data not shown) were quantified (data not shown). Those calculations will be performed to estimate the size of B cell repertoire (see below).

**Example 4: Estimation of the size of peripheral blood IgM+ and total B lymphocytes repertoires**

To estimate the global size of the B cell repertoire, the inventors first focused on the VH5-JH2 rearrangement in donor 789 for the following reasons. First, VH5 family only includes two germline genes and its overall usage in the constitution of the antibody repertoire is not more then 3.2% (see Table 3). Second, JH2 segment is the second less utilized gene segment among all JH fragments (2,1% of all VH5 rearrangements) (Table 3). Therefore, it can be hypothesised that, after Immunoscope separation and quantification of all CDR3 peaks, the exhaustive sequencing of those rearrangements will be feasible. Third, the Immunoscope profile of VH5-JH2 rearrangement in donor 789 is representative of most blood B cell rearrangements, characterised by a quasi-Gaussian repartition only slightly perturbed by the presence of few clonal expansions (Figure 1B). Finally, the utilisation of VH5-JH2 rearrangement to base B cells repertoire size calculations is possible due to the random utilisation of the variable genes in the adult.
The first objective was to study the general size of the B cell repertoire irrespectively of yhe isotyp expressed by the antibody produced. To achieve this goal, cDNA from donor 789 was PCR amplified using VH5 gene family-specific primer together with IGJH2 specific primer (see Table 2). « Run off » reaction was then performed with a JH2-Fam fluorescent probe and the specific Immunoscope profile obtained (Figure 1B). Since this profile includes VH5-JH2 rearrangements for all antibody isotypes, it can be considered to be representative of the total size of the repertoire. Indeed, the total number of individual sequences included in this profile is beyond attempt of entire VH5-JH2 rearrangement exhaustive sequencing. Consequently, as previously described (Casrouge and al. (2000). J Immunol 164, 5782-7), the band corresponding to a CDR3 length of 8 amino acids was cut from the polyacrylamide gel and subjected to a second PCR using the VH5int and IGJH2 primers (Table 2). Because the diversity found in the CDR3 of this band is proportional to the peak aera deduced from the Immunoscope profile, the size of total repertoire can be deduced from exhaustive sequencing of the band. This sequencing is now made possible since only a known fraction of all the VH5-JH2 rearrangement will be considerered. Exhaustive sequencing of a given purified CDR3 band or a total rearrangement is considered to be achieved when the total number of different sequences versus the total number of clones sequenced reach a plateau (Arstila and al. (1999). Science 286, 958-61).Table 4 show that the plateau in the number of different sequences is reached for 72 different sequences for the 8aa band. Aglobal CDR3 diversity of 5.8 $10^6$ can be calculated from the 8aa band dividing the number of sequences by the product of the percentage of usage of VH5, the percentage of usage of JH2 and the percentage of the 8aa band among VH5-JH2 rearrangement. Similar results are obtained when other CDR3 bands are purified (data not shown).

As IgM positive B cells are by far the most frequent subset among PBMC (Table 1), the inventors then focused on the determination of repertoire size of these cells. For this purpose, the inventors decided to concentrate on VH5-JH1 rearrangement because on one hand, JH1 gene segment is the less utilized among all JH fragments (0.2%) (Table 3) and on the other hand exhaustive sequencing was possible on the whole VH5-JH1 rearrangement. In this respect, cDNA from donor 789 was PCR amplified using the VH gene family and the Cμ specific primers, followed by "run-off" reaction using a VH5-Fam fluorescent probe (Table 2). The specific VH5-JH1 immunoscope profile is shown in Figure 1B. The inventors succeeded in the exhaustive sequencing of VH5-JH1 rearrangement of donor 789, although a total number of one thousand sequences had to be performed to reach a plateau of 346 different sequences (Table 4). A global CDR3 diversity of 5.1 $10^6$ was evaluated for the IgM bearing B cells by dividing the number of different sequences by the product of the percentage of usage of VH5 and the percentage of usage of JH1. This number is close to the size of the total repertoire, consistent with the prevalence of IgM positive B cells among PBMC.

To confirm this finding and to valididate of this approach, the inventors performed global sequencing of the VH5-JH1 rearrangement amplified from donor 743, and this was done twice on the two samples W and Z, which harbor the same number of IgM positive B cells (2.7 $10^6$ i.e. 1/6 of the total amount of purified B cell). The specific VH5-JH1 immunoscope is shown in Figure 1B for both samples. Exhaustive sequencing of 655 and 675 total sequences was performed for sample W and Z respectively, giving 232 and 228 different sequences respectively (Table 4). The size of the IgM repertoire was then calculated as described above, leading to a CDR3 diversity of 2.1 x $10^6$ and 2.4x$10^6$ in samples W and Z respectively. Consequentely, diversity calculation for the 500 ml blood sample from donor 743 is equal to (W+Z)x3 = 1.3x$10^7$ (Table 4).

**Example 5: Contribution of somatic mutations to diversity and determination of the general clone size of peripheral B cells**

As compared with T cells, one of the most striking difference in generating diversity in B cells is the property to accumulate somatic mutations in their variable regions. Somatic mutations are supposed to be generated mainly in the germinal center, as the key phenomenon in affinity maturation and maintenance of memory (Wu and al. (2003). J Clin Immunol 23, 235-46). Somatic mutations are not restricted to IgG expressing B cells since 35% of human blood IgM+B cells have been reported to display somatic mutations in heavy chain variable genes (Klein and al. (1997). Blood 89, 1288-98). The calculations described above to define the size of the B cells repertoire take into consideration CDR3 diversity, but not the diversity generated by somatic mutations. The inventors studied first these mutations that were detected in two clones during the exhaustive sequencing of the VH5-JH2 and VH5-JH1 rearrangements. By definition, the inventors consider that belong to the same clone all the sequences bearing the same CDR3. Using this criteria, it is possible to draw phylogenic trees of the mutations. Clone A originates from the sequencing of VH5-JH2 rearrangement from donor 789, it bears a CDR3 sequence of 14 aminoacids, it expresses an IgM receptor and has been found 67 times. Clone B originates from the sequencing of VH5-JH1 rearrangement from donor 743, it bears a CDR3 sequence of 25 aminoacids, i expresses an IgM receptor and have been found in 130 sequences. Both clone A and B mutations trees are shown in Figure 2. These two clones display very different patterns of mutations. For clone A, only 3 sequences are found in germline configuration and the vast majority of sub-clones are the ones that accumulate the highest numbers of mutations (sub-clones G, I and L). Most of those mutations are related and can be deduced from a linear tree. Out of 21 codons subjected to mutations, only 3 give rise to silent mutations. On the other hand, clone B displays 33 sequences without mutations and many sub-clones bearing non related mutations could be directly deduced from the germline sequence, leading to a "star" pattern of mutations. Only sub-clones bearing few number of mutations show a large accumulation (sub-clone N). Out of 35 codons subjected to mutations, 10 gave rise to silent mutations.

If those two examples of clonal mutations can be informative in the follow-up of a given immune response, they are not significant enought to evaluate the contribution of somatic mutations to the diversity. In order to calculate the global contribution of somatic mutations to diversity, the inventors have compared all the VH sequences obtained from exhaustive sequencing of the different rearrangements to the germline VH irrespectively of the CDR3 sequences. Results are shown in Table4. For both donors and for both VH5-JH2 and VH5-JH1 rearrangements, approximately half of the VH sequences are found bearing one or several somatic mutations. Taking into account this B cell specific additional source of diversity, the global repertoire size of the studied blood samples can be estimated (Table 4). For donor 789, the value obtained for 500ml blood sample ranges from 6.7 x $10^6$ to $10^7$ depending of the rearrangement studied, the methodology used (with or without band purification), and the specificity of the PCR amplification (all isotypes or only IgM). Taking into account a degree of uncertainty inherent to the approach used, the VH repertoire of B cell repertoire is around one order of magnitude larger than the Vβ T cells repertoire (Arstila and al. (1999). Science 286, 958-61).

This result has other implications in what concern the properties of the B cells repertoire. The estimated size of the B cell repertoire, calculated from a given number of B cells, is always very close to this number and this was even more manifest when only a small number of B cells was studied, as in W and Z samples from donor 743. In this case, the repertoire number obtained was even slightly larger than the number of B cells, but still included the confidence limits interval (see Table 3). In other words, in a 500 ml blood poach, according to the diversity of their VH genes repertoire, each B cells express and produce a different antibody, with the exception of antigen specific clonal expansions. Consequently, assuming that the total amount of blood in one given individual is close to 5 liters, the global B cells clone size must be comprised between 10 and 1 and therefore the diversity of B cell repertoire must range accordingly between $10^7$ and $10^8$. Again, this result strengthens the differences existing between T cells and B cells repertoire as the T cell Vβ clone size has been reported to be approximatively equal to 100 (Arstila and al. (1999). Science 286, 958-61).

**Example 6: Determining the proportion of clonal expansions within one given VDJ rearrangement**

Another property of B cells is the capability of developing very large clonal expansions in response to antigenic stimulation. Clonal expansion can be defined according to different criteria : 1°), it corresponds to all sequences sharing the same CDR3 sequence, irrespective on the presence or not of somatic mutations in their V regions. The inventors have used this definition to represent trees of mutations for a given clone (see Figure 2). In this case, all sequences sharing complete identity toward all VH region in addition to the CDR3 could be generated by exhaustive sequencing. 2°) it correspond to all sequences sharing identical CDR3 sequences found for a given rearrangement. 3°) clonal expansions might also be taken in consideration above a certain threshold of representation. In order to address this issue, the inventors have performed exhaustive sequencing of the VH5-JH1 rearrangement from two samples originated from donor 743, containing the same number of purified B cells. As shown in Figure 3, three differents situations can be distinguished depending in the number of copies a given sequences can be found. If the number of copie per sequence is less than 5, which represent around 70% of total sequences, there is only 5 % of overlaping sequences in between samples W and Z, constituted by 3% of mutated sequences and 2% of non mutated sequences. Similarely, non commun sequences between samples W and Z are almost equaly split in mutated and non mutated sequences (48% versus 47%) (Figure 3B). When the copie number per sequence ranged between 6 and 10, the non common germline sequences present in W or Z still account for half of the sequence (52%) but shared W and Z sequences have increased to 24% (see Figure 3B). This intermediate situation, which will be representative of « small clones» represent 12% of the total W&Z sequences. Finally, a population of « large clones » with a copie number per sequences higher than 10 and representing 18% of total sequences, are found. In this case, the majority of sequences (73%) are common W and Z sequences and most of then habor mutations (64%). Therefore, depending if small clones are taken in consideration or not, the proportion for clonal expansions ranged between 18% and 30% of all VH5-JH1 rearrangement sequences from donor 743. A similar proportion of clonal expansions were found from VH5-JH1 and VH5-JH2 rearrangements from donor 749 (Data not shown). As already mentioned above, if clonal expansions are not taken in consideration, 95% of the sequences are found in sample W or Sample Z but not in both. This finding strengthen evidences for a small global clone size.

**DISCUSSION**

**1°) The diversity of the Immunoglobulin VH genes repertoire is comprised between $10^7$ and $10^8$ different expressed rearrangements**

In this study, the inventors report the first size estimate of the human B cells VH gene repertoire. To reach this goal, the inventors first quantified by real time PCR VH gene utilisation in total peripheral B cells isolated from normal donors. The inventors then focus on the VH5 gene family which display an intermediate rate of utilisation and is composed by only few germline genes. JH gene segment utilisation was then quantified in all VH5 gene family rearrangements. Two rearrangements, VH5-JH1 and VH5-JH2, were chosen and extensively studied. In the case of VH5-JH1 rearrangement, Immunoscope was performed in order to separate and quantify the representation of 8 and 9 amminoacids long CDR3. The corresponding bands were then purified, cloned and subjected to exhaustive sequencing. Calculations have allowed to estimate the size of the VH gene repertoire. For the VH5-JH2 rearrangement, exhaustive sequencing was performed on the total PCR product, for all the different CDR3 lengths. In both case, the estimate of the VH gene global diversity is close to $10^7$ for all B cells purified from 500ml of human blood. If the total amount of blood of a normal donor is roughly equal to 5 liters, the global diversity of the human peripheral B cell VH gene repertoire must be close to $10^8$ expressed rearrangements

**2°) The mean global clone size of peripheral B cells is close to 1**

From the above result on global diversity of the human peripheral B cell, it can be estimated that the global clone size of immunoglobulin expressing cells must varied between 1 and 10. As a matter of fact, when separated sequencing is performed on VH5-JH2 rearrangement from two samples of the same donor harboring the same number of cells, the size of the repertoire calculated in both case is almost identical to this number of cells. The same result was obtained with different number of cell in the studied sample, even when the number of cells is equal to the B cells total number of the blood gift as for the sequencing of VH5-JH1 rearrangement .In addition, a detailled study of the sequences of each of this two samples show that, with the exception of large clonal expansions with mutated VH genes, not more than 5% of the sequences are found in both samples. Therefore, if B cell diversity from a blood gift can be estimated to reach $10^7$ different VH and if the mean global clone size for B cell is close to 1, the total VH diversity must reach $10^8$. Alternatively, if B cell mean global clone sze is closer to 10, the overall size of the VH repertoire of the donor should be similar to that of the blood gift, ie $10^7$.

**3°) VH-VL pairing is poorly involved the generation of B cells diversity**

If the calculation of the diversty of VH repertoire always give a number similar to that of the number of cells studied, one can assume that VH genes diversity can be similar to B cells diversity. On the contrary than for the T cells repertoire, VL, repertoire and VH-VL pairing are less involved in the diversity of the B cells repertoire than Vα repertoire and Vα-Vβ paring in the diversity of T cells repertoire. If each B cells in the periphery which cannot be considered to belong to a clonal expansion (and therefore representing at least.70% of all B cells) is already identify simply according to its VH usage, size determination of the VL repertoire can be assumed to provide similar values than for VH calculations. In other words, if VH rearrangement is enought by itsef to define a given B cell, each B cell expressing a particular VH-VL pair is necessarily single. This situation is completely different to what have been already reported for the T cells repertoire, where each Vβ must pair in average with 25 different α chain leading to a total αβ TCR diversity in the blood not less than 25x $10^6$ differents TCR. Therefore, if both T and B cells repertoire reach approximatively the same size of repertoire (between $10^7$ and $10^8$ different expressed rearrangements), the way by which this level of diversity is achieved strongly differ for each cell type. In B cells, the size of the repertoire mainly rely on VH CDR3 diversity and on VH somatic mutations. Consecutively, the size of the VH repertoire is at least one order of magnitude larger as compared to the Vβ repertoire. On the contrary, in addition to Vβ and Vα diversity, T cells repertoire diversity result also in the different possibilities of pairing between Vα and Vβ chains. The importance of Vα-Vβ pairing in the T cells repertoire diversity have been postulated to result in part from several rounds of cell divisions between Vβ and Vα rearrangements, allowing the same Vβ to pair with differents Vα. This rise the possibility that VL rearrangements could occur in B cells shortly after VH rearrangements within a window of time allowing less cell divisions than for T cells, preventing a B cell clone expressing a given VH to expand as much as a T cell clone. Evidences showing that, in the mouse, VL rearrangements can even occur prior to VH rearrangements are also in line with this hypothesis. The importance of Vα-Vβ pairing in the T cell repertoire diversity can also derived from thymocyte positive and negative selection in the thymus. The result of this process lead to the induction of apoptosis for a large majority of cells and selection of a small number according to the specificity of their TCR. Therefore, blood circulating T might be more representative of a Vα-Vβ pair selected population while blood circulating B cells, with the exception of clonal expansions, could be more closely related to the bone marrow outcome. For the T cells, the selection of the Vα-Vβ pair occur in the thymus on the basis of MHC recognition, and allow the local expansion of the cells expressing the selected TCR. Indeed, the mean clone size for T cell have been estimated to reach 100 copies in the periphery. For B cells, on the contrary, our results show a much smaller clone size, ranging between I and 10. Another hypothesis to explain this difference, would be to postulate that bone marrow produce B cells with fewer constrainsts of selection than for T cells in the thymus. Indeed, two major steps of selection of B cells have been reported to occur in the bone marrow. First, once a functional VDJ rearrangement is acheived and an μ heavy chain expressed at the surface of the pre-B cell, failure of pairing with the VpreB lambda5 surrogate light chain could prevent further differentiation. Second, cells expressing a self reactive BCR have been described to modify their receptor specificity by a process denominated « receptor editing ». Nevertheless, the properties of BCR antigen recognition, leading to the ability to identify a three dimentional patem, differ strongly with the TCR capacity to recognize a given peptide in the context of a given MHC. In addition, our results being consistant with no or few antigen independent cell divisions in the bone marrow, its outcome could be more representative of a random distribution of BCR than the thymus TCR outcome. Indeed, being deduced from total peripheral B cells, our results do not allow the complete assimilation of this population with the bone marrow outcome. Furthermore, in the mouse, peripheral B cells have been described to be mainly ligand selected. In order to gain some insights on this issue, studies on purified B cell populations representative of the recent bone marrow emigrant should be informative.

**4°) Clonal expansions account for 20 to 30% of a given rearrangement**

Over than 15 years ago, Langman and Cohn have proposed, based on informatic stimulations and on B cells repertoire observations that B cell repertoire could be divided in several identical sub-fractions, each sub-fraction being a « functional unit » of $10^7$ B lymphocytes bearing a repertoire of $10^5$ rearrangements (Langman and Cohn, 1987). Our findings that the global B cell clone size must be be comprise between one and ten and that most peripheral B cells are unique are apparently incompatible with the protecton theory which postulate, in organims haboring more than $10^7$ lymphocytes, a important redandundancy of BCR specificities. Paradoxicaly, T cells repertoire properties, as they were previously described (Arstila et Wagner) seems more compatible with a direct interpretation of the protecton theory. First, as already mentionned by Casrouge et al, the difference of T cells diversity between mice [Casrouge and al. (2000). J Immunol 164, 5782-7] and human [Arstila and al. (1999). Science 286, 958-61]does not reflect the much larger difference in total number of peripheral T lymphocytes between these two species. Second, T cells global clone size appear, in particular in human, to be much larger than B cells one and therefore being compatible with the existence of several functional units.

Another property of the B cells repertoire hardly seems compatible with our results. In both mice and human, B cells produce « natural antibodies » which are mainly low affinity IgM antibodies, usualy polyspecific and germ line encoded. Those antibodies, which are predominant in newborn life, often recognise self antigen. Their production is constant over life period and very stable, antibodies against given specificities always being found in differents individuals. How could this property of the B cells repertoire could be achieved if almost each peripheral B cell habor a unique specificity

In order to reconcile our findings with both protecton hypothesis and production of natural antibodies, the inventors wishes to propound the following hypothesis. The maintenance of natural antibody production could be achieved, not by a continuous bone marrow outcome, but rather by continously activated peripheral B cells, self ligand selected and eventually giving rise to clonal expansions. Similarely, protecton theory could only concern the pool of clonal expansions among all peripheral B cells. In this case, the stability of natural antibodies production will be ensure mainly by peripheral proliferation of clonal expansions, which represent, according to our results, up to 30% of the total number of sequences for one given rearrangement. As illustrated in the two examples of clonal expansions described in Fig 2, some sequences belonging to clonal expansions are in germline configuration (3 for clonal expansion A, 33 for clonal expansion B), as expected for natural antibody producing clones. Some clonal expansions would also account for external antigen driven proliferations. The occurrence of mutations should therefore not only allow affinity maturation of the B cell repertoire but also help to diversify the natural antibody production. Indeed, in accordance with what have been determined by single cell PCR, the inventors found that up to 40% of IgM positive B cells in the blood have mutated VH.

The results provide therefore, in addition to the first determination of the size of the peripheral B cells repertoire, some hypothesis for a better understanding of the mechanisms involved in the maintenance of this repertoire. The quantitative approach developped here could also be a powerfull tool to study B cells repertoire disturbancies occuring during pathological and autoimmune diseases.

**Table 1: Proportion and absolute numbers of cells expressing different isotype in the blood of two healthy donors**

Blood cells from donor 743 and 522 were stained before and after B cell enrishment with the different antibodies according to the materiel and method and analysed on a FACScalibur (Becton Dickinson). For each staining, result are expressed in % of total cells and corresponding absolute numbers are shown in parenthesis.

**<u>Table 1</u> : Proportion and absolute number of cells expressing different isotype in the blood of two healthy donors.**

| | Donor 743 | | Donor 522 | |
|---|---|---|---|---|
| Type of positive cells Among **all cells** | Non Purified Fraction **%(Absolute nb x $10^6$)** | CD19 Purified Fraction **%(Absolute nb x $10^6$)** | Non Purified Fraction **%(Absolute nb x $10^6$)** | CD19 Purified Fraction **%(Absolute nb × $10^6$)** |
| CD 19 | 3,8 **(19,8)** | | 3,7 **(17,8)** | 67,4 **(14,2)** |
| IgM | 3,2 **(16,7)** | 64,5 **(16,3)** | 2,3 **(11)** | 42,3 **(8,9)** |
| IgD | 2,6 **(13,5)** | 49,6 **(12,5)** | 2,3 **(11)** | 51 **(10,8)** |
| IgG | 0,6 **(3,1)** | 12 **(3)** | 0,2 **(1)** | 4,6 **(2,1)** |
| IgA | 0,8 **(4,2)** | 16,1 **(4,1)** | 0,5 **(2,4)** | 10,1 **(2,1)** |
| IgE | 1 **(5,2)** | 1,1 **(0,3)** | 0,3 **(1,4)** | 1,3 **(0,3)** |
| Kappa | **4 (20,8)** | 44,2 **(11,1)** | 2,8 **(13,4)** | 41,9 **(8,8)** |
| Lambda | 3,3 **(17,2)** | 40,1 **(10,1)** | 2,3 **(11)** | 29,8 **(6,3)** |
| Sum Kappa & Lambda | 7,3 **(38)** | 84,3 **(21,2)** | 5,1 **(24,4)** | 71,7 **(15,1)** |

(continued)

| | Donor 743 | | Donor 522 | |
|---|---|---|---|---|
| Type of positive cells Among **all cells** | Non Purified Fraction **%(Absolute nb x $10^6$)** | CD19 Purified Fraction **%(Absolute nb x $10^6$)** | Non Purified Fraction **%(Absolute nb x $10^6$)** | CD19 Purified Fraction **%(Absolute nb × $10^6$)** |
| Kappa & Lambda | | | 4,8 **(23)** | 68,4 **(14,4)** |
| Total Cell Number | 520 | 25,2 | 480 | 21 |

**Table 2: Specific primers for B cell heavy chain quantitative Immunoscope analysis**

This table shows the list of primers utilized to quantified VH and JH utilization by real time PCR. Each primers specificaly amplify one or several VH, as notified. The primers localisation in the FR1 or FR3 is also shown. 3' end phosphorothioate chemical modification of some primers is mentionned by a *. Only two primers display degenerate sites with R meaning A or G and S meaning G or C.

**Table 2 : List of specific primers for B cell heavy chains quantitative Immunoscope analysis.**

| | Primer name: | Sequence: | Specificlty : | Primer localisation |
|---|---|---|---|---|
| **IGVH subgroup** | | | | |
| VH1 | HUMVH1a | AGTGAAGGTCTCCTGCAAGGC | VH1-02,06,18,58,69,e | FR1 |
| | HUMVH1b | AGTGAAGGTTTCCTGCAAGGC | VH1-03,45.46 | FR1 |
| | HUMVH1c | AGTGAARRTCTCCTGCAAGGT | VH1-f,24 | FR1 |
| VH2 | HUMVH2 | AACCCACASAGACCCTCAC | VH2-05,70,26 | FR1 |
| VH3a | HUMVH3aa | GCAGATTCACCATCTCAAGAGATG | VH3-15,49,72 | FR3 |
| | HUMVH3ab | GCAGGTTCACCATCTCCAGAGATG | VH3 -73 | FR3 |
| VH3b | HUMVH3ba | GCCGATTCACCATCTCCAGAGA | VH3-07,09,13,20,21,30,30.3 33,43,48,53,74 | FR3 |
| | HUMVH3bb | GCAGATTCACCATCTCCAGAGA | VH3-d,64,66 | FR3 |
| | HUMVH3bc | GCCGATTCACCATCTCCAGGGA | VH3-11 | FR3 |
| | HUMVH3bd | GCAGGTTCACCATCTCCAGAGA | VH3-23 | FR3 |
| VH4 | HUMVH4a | CTACAACCCGTCCCTCAAGAGT | VH4-04,28,30-2,30-4,31,34,b | FR3 |
| | HUMVH4b | CTACAACCCCTCCCTCAAGAGT | VH4-59,61 | FR3 |
| VH5 | HUMVH5 | GTGAAAAAGCCCGGGGAG | VH5-51,a | **FR1** |
| VH6 | HUMVH6 | TCCGGGGACAGTGTCTCT | VH6-01 | **FR1** |
| VH7 | HUMVH7 | GGTGCAATCTGGGTCTGAGT*T | VH7-04.1 | FR1 |
| **IGJH subgroup** | | | | |
| JH1 | IGJH1 | CCCTGGCCCCAGTGCT*G | JH1 | |
| JH2 | IGJH2 | CCACGGCCCCAGAGATC*G | JH2 | |
| JH3 | IGJH3 | CCCTTGGCCCCAGAYATCAAAA*G | JH3a,b | |
| JH4 | IGJH4.1 | GGTTCCTTGGCCCCAGTA*G | JH4a | |
| | IGJH4.2 | GGTTCCCTGGCCCCAGTA*G | JH4b | |
| | IGJH4.3 | GGTCCCTTGGCCCCAGTA*G | JH4d | |
| JH5 | IGJH5 | TGGCCCCAGG RGTCGAA*C | JH5a,b | |
| JH6 | IGJH6.1 | CCTTGCCCCCAGACGTCCA*T | JH6a | |
| | IGJH6.2 | CCTTGGCCCCAGACGTCCA*T | JH6b | |
| | IGJH6.3 | CCTTTGCCCCAGACGTCCA*T | JH6c | |
| **IGH mu chain** | | | | |
| | HIGCM | CAGCCAACGGCCACGC | IGHM.01,02,03 | CH1 |
| | HCM-Fam | 6Fam-GGAGACGAGGGGGAAAAGG | | CH1 |
| | HCM-MGB | 6Fam-CCGTCGGATACGAGC-MGB | | CH1 |

**Table 3: $V_{11}$ and $J_H$ quantification by real time PCR**

Table 3 A shows the mean percentage of VH utilisation determined by real time PCR for B cells from donor 789 and 743. For donor 743 , this determination was done separately on the two samples W and Z, as displayed. Table 3B shows the JH segment usage in association with the VH5 gene family.

**Table 3 : $V_H$ and $J_H$ quantification by real time PCR**

**A**

| | Donor 789 | Donor 743 | |
|---|---|---|---|
| | | Sample W | Sample Z |
| VH Families | %Means | % | % |
| VH1 | 5,9±1 | 4,9 | 4,5 |
| VH2 | 9,5 ± 1,5 | 4,7 | 4,4 |
| VH3a | 5,8 ± 0,1 | 6,7 | 7,3 |
| VH3b | 52,5 ± 2,1 | 65,7 | 65,5 |
| VH4 | 20,2 ± 1,4 | 13,2 | 13,3 |
| VH5 | 3,2 ± 0,3 | 1,1 | 1 |
| VH6 | 2,6 ± 0,9 | 1,3 | 1,3 |
| VH7 | 0,3 ± 0,2 | 2,5 | 2,8 |

**B**

| VH5 | Donor 789 | Donor 7 43 | |
|---|---|---|---|
| | | sample W | sample Z |
| JH Families | % Means | % | % |
| JH1 | 0,2 ± 0,05 | 1 ± 0,2 | 1 ± 0,1 |
| JH2 | 2,1 ± 0,4 | 2,5 ± 0,1 | 2,9 ± 0,5 |
| JH3 | 10,2 ± 0,5 | 14,1 ± 2,2 | 15,5 ± 1,2 |
| JH4 | 41 ± 2,3 | 42,1 ± 1,8 | 40,7 ± 1 |
| JH5 | 34,6 ± 2 | 16,1 ± 0,8 | 18,5 ± 1 ,8 |
| JH6 | 11,9 ± 0,8 | 24,2 ± 0,8 | 21,4 ± 1,3 |

**Table 4 : Global estimate of B cells diversity.**

| | **Donor 789** | | **Donor 743** | |
|---|---|---|---|---|
| Rearrangement | VH5-JH2 | VH5-JH1-CM | VH5-JH1-CM | |
| | | | sample W* | sample Z* |
| Amplified isotype | All | IgM | IgM | |
| CDR3 length | 8 aa | All peaks | Al peak s | |
| Number of B cells after purification ($x10^6$) | 21,6 | | 3,5 | |
| Number of $IgM^+$ cells ($x 10^6$) | 10,8 | | 2,3 | |
| Total number of sequences analysed | 261 | 981 | 655 | 675 |
| Number of different sequences | 72 | 346 | 232 | 228 |
| Size of CDR3 repertoire ($x 10^6$) | 5,8 | 5,1 | 2,1 | 2,4 |
| Frequency of mutated seq | 42% | 24% | 38% | 30% |
| Size of sample total repertoire ($x 10^6$) | 10 | 6,7 | 3,4 | |
| Clone size | ~1 | | ~1 | |

## Claims

**1.** A process for determining the quantitative and qualitative profile of the repertoire of a given type of an immunoglobulin heavy chain expressed by a lymphocyte B population present in a tissue sample, **characterised in that** it comprises the following steps:

(a) obtaining either the cDNA from the mRNA expressed from the tissue sample or the cellular DNA extract of the tissue sample,
(b) performing the amplification of the cDNA obtained at the step (a) with a set of VH forward primers capable of specifically hybridizing in stringent conditions with the nucleic acids encoding the variable segments (VH) of immunoglobulin heavy chains, said variable segments being distributed among VH subgroups, associated with a CH reverse primer, or a mixture thereof, capable of specifically hybridizing in stringent conditions with the nucleic acid encoding the constant segment (CH) of a given type of an immunoglobulin heavy chain, and
(c) determining the quantitative and qualitative profile of the repertoire of said type of immunoglobulin heavy chain for each VH subgroup,

**characterized in that** the set of VH forward primers comprises at least the 8 following subgroups of VH primers corresponding to the VH subgroups :

- the VH1 primers having the sequences SEQ ID N° 1 to SEQ ID N° 3, and
- the VH2 primer having the sequence SEQ ID N° 4, and
- the VH3a primers having the sequences SEQ ID N° 5 and SEQ ID N° 6, and
- the VH3b primers having the sequences SEQ ID N° 7 to SEQ ID N° 10, and
- the VH4 primers having the sequences SEQ ID N° 11 and SEQ ID N° 12, and
- the VH5 primer having the sequence SEQ ID N° 13, and
- the VH6 primer having the sequence SEQ ID N° 14, and
- the VH7 primer having the sequence SEQ ID N° 15.

2. The process for determining the quantitative and qualitative profile according to claim 1, **characterized in that** separated amplifications are performed for each of the VH subgroups.

3. The process for determining the quantitative and qualitative profile according to claim 2, **characterized in that** the separated amplifications are real-time separated amplifications, said real-time amplifications being performed using a CH labeled reverse probe, preferably a CH labeled reverse hydrolysis-probe, capable of specifically hybridizing in stringent conditions with the constant segment of the given type of immunoglobulin heavy chain and capable of emiting a detectable signal everytime each amplification cycle occurs, and **characterized in that** the signal obtained for each VH subgroup is measured.

4. The process for determining the quantitative and qualitative profile according to claim 2 or 3, **characterized in that** the separated amplification products obtained for each of the VH subgroups are further elongated using a CH labeled reverse probe capable of specifically hybridizing in stringent conditions with the constant segment of the given type of immunoglobulin heavy chain and capable of emiting a detectable signal, and **characterized in that** the elongation products are separated, for each of the VH subgroups, relative to their length, the signal obtained for the separated elongation products is measured, and the quantitative and qualitative profile of the labeling intensity relative to the elongation product length is established, for each of the VH subgroups individually.

5. The process for determining the quantitative and qualitative profile according to anyone of claims 1 to 4, **characterized in that** the sequences SEQ ID N° 1 to SEQ ID N° 15 may contain at least one to three point mutations, except for the nucleotides 1 to 6 of their 3' part.

6. The process for determining the quantitative and qualitative profile according to claim 1 to 5, **characterized in that** the CH reverse primer is selected from the CH reverse primers capable of specifically hybridizing in stringent conditions with the nucleic acids encoding the constant segments (CH) of the IgM heavy chain, the IgE heavy chain and the IgA heavy chain.

7. The process for determining the quantitative and qualitative profile according to claim 6, **characterized in that**, when the CH reverse primer is capable of specifically hybridizing in stringent conditions with the nucleic acid encoding the constant segment (CH) of the IgM heavy chain, the CH reverse primer has the sequence SEQ ID N° 26, or the sequence SEQ ID N° 26 wherein one to three point mutations may occur, except for the nucleotides 1 to 6 of its 3' part.

8. The process for determining the quantitative and qualitative profile according to claim 6, **characterized in that**, when the CH reverse primer is capable of specifically hybridizing in stringent conditions with the nucleic acid encoding the constant segment (CH) of the IgE heavy chain, the CH reverse primer has the sequence SEQ ID N° 33, or the sequence SEQ ID N° 33 wherein one to three point mutations may occur, except for the nucleotides 1 to 6 of its 3' part.

**9.** The process for determining the quantitative and qualitative profile according to anyone of claims 1 to 5, **characterized in that**, when the given type of immunoglobulin heavy chain is the IgG type, a mixture of two CH reverse primers is associated with the set of VH forward primers, said two CH reverse primers having the sequences SEQ ID N° 27 and SEQ ID N° 28, or the sequences SEQ ID N° 27 and SEQ ID N° 28 wherein one to three point mutations may occur, except for the nucleotides 1 to 6 of its 3' part.

**10.** The process for determining the quantitative and qualitative profile according to claim 6 or 7, **characterized in that**, when the given type of immunoglobulin heavy chain is an IgM heavy chain and when the separated amplifications are real-time separated amplifications, the CH labeled hydrolysis-probe has the sequence SEQ ID N° 29, or the sequence SEQ ID N° 29 wherein one or two point mutations may occur.

**11.** The process for determining the quantitative and qualitative profile according to claim 6 or 7, **characterized in that**, when the given type of immunoglobulin heavy chain is an IgM heavy chain and when the separated amplification products obtained for each of the VH subgroups are further elongated, the CH labeled reverse probe has the sequence SEQ ID N° 30, or the sequence SEQ ID N° 30 wherein one to three point mutations may occur, except for the nucleotides 1 to 6 of its 3' part.

**12.** The process for determining the quantitative and qualitative profile according to claim 6 or 8, **characterized in that**, when the given type of immunoglobulin heavy chain is an IgE heavy chain and when the separated amplifications are real-time separated amplifications, the CH labeled hydrolysis-probe has the sequence SEQ ID N° 36, or the sequence SEQ ID N° 36 wherein one or two point mutations may occur.

**13.** The process for determining the quantitative and qualitative profile according to claim 6 or 8, **characterized in that**, when the given type of immunoglobulin heavy chain is an IgE heavy chain and when the separated amplification products obtained for each of the VH subgroups are further elongated, the CH labeled reverse probe has the sequence SEQ ID N° 37, or the sequence SEQ ID N° 37 wherein one to three point mutations may occur, except for the nucleotides 1 to 6 of its 3' part.

**14.** The process for determining the quantitative and qualitative profile according to claim 9, **characterized in that**, when the given type of immunoglobulin heavy chain is an IgG heavy chain and when the separated amplifications are real-time separated amplifications, the CH labeled hydrolysis-probe has the sequence SEQ ID N° 34, or the sequence SEQ ID N° 34 wherein one or two point mutations may occur.

**15.** The process for determining the quantitative and qualitative profile according to claim 9, **characterized in that**, when the given type of immunoglobulin heavy chain is an IgG heavy chain and when the separated amplification products obtained for each of the VH subgroups are further elongated, the CH labeled reverse probe has the sequence SEQ ID N° 35, or the sequence SEQ ID N° 35 wherein one to three point mutations may occur, except for the nucleotides 1 to 6 of its 3' part.

**16.** The process for determining the quantitative and qualitative profile according to anyone of claims 2 to 15, **characterized in that** further separated amplifications for each of JH subgroups are performed from the separated amplification products obtained for at least one given VH subgroup of the VH subgroups with the CH reverse primer, said further separated amplifications being performed using a VH internal forward primer corresponding to the given VH subgroup, and associated with a set of JH reverse primers corresponding to the JH subgroups and capable of specifically hybridizing in stringent conditions with the nucleic acids encoding the junction segments of the given type of immunoglobulin heavy chain.

**17.** The process for determining the quantitative and qualitative profile according to claim 16, **characterized in that** the further separated amplifications are real-time amplifications performed using a VH labeled forward probe, preferably a VH labeled forward hydrolysis-probe, capable of specifically hybridizing in stringent conditions with the variable segment of the given type of immunoglobulin heavy chain and capable of emiting a detectable signal everytime each amplification cycle occurs, and **characterized in that** the signal obtained for each JH subgroup is measured.

**18.** The process for determining the quantitative and qualitative profile according to claims 16 or 17, **characterized in that**, when the given VH subgroup is the VH5 subgroup, the VH5 internal forward primer has the sequence SEQ ID N° 31, or the sequence SEQ ID N° 31 wherein one to three point mutations may occur, except for the nucleotides 1 to 6 of its 3' part.

**19.** The process for determining the quantitative and qualitative profile according to claim 18, **characterized in that** the VH labeled forward hydrolysis-probe has the sequence SEQ ID N° 32, or the sequence SEQ ID N° 32 wherein one or two point mutations may occur.

**20.** The process for determining the quantitative and qualitative profile according to anyone of claims 2 to 15, **characterized in that** separated elongations are performed for each of the JH subgroups from the separated amplification products obtained for at least one given VH subgroup of the VH subgroups with the CH reverse primer,
said further separated elongations being performed using a set of JH labeled reverse primers corresponding to JH subgroups and capable of specifically hybridizing in stringent conditions with the nucleic acids encoding the junction segments of the given type of immunoglobulin heavy chain, said JH labeled reverse primers being capable of emiting a detectable signal,
and **characterized in that** the elongation products are separated, for each of the JH subgroups, relative to their length, the signal obtained for the separated elongation products is measured, and the quantitative and qualitative profile of the labeling intensity relative to the elongation product length is established, for each of the JH subgroups for the given VH subgroup.

**21.** The process for determining the quantitative and qualitative profile according to anyone of claims 16 to 20, **characterized in that** the set of JH forward primers, optionally labeled, comprises at least the 6 following subgroups of JH primers corresponding to the JH subgroups :

- the JH 1 primer having the sequence SEQ ID N° 16, and
- the JH2 primer having the sequence SEQ ID N° 17, and
- the JH3 primer having the sequence SEQ ID N° 18, and
- the JH4 primers having the sequences SEQ ID N° 19 to SEQ ID N° 21, and
- the JH5 primer having the sequence SEQ ID N° 22, and
- the JH6 primers having the sequences SEQ ID N° 23 to SEQ ID N° 25.

**22.** The process for determining the quantitative and qualitative profile according to claim 21, **characterized in that** the sequences SEQ ID N° 16 to SEQ ID N° 25 may contain at least one to three point mutations, except for the nucleotides 1 to 6 of their 3' part.

**23.** A set of VH forward primers capable of specifically hybridizing in stringent conditions with the nucleic acids encoding the variable segments (VH) of immunoglobulin heavy chains, said variable segments being distributed among at least 8 VH subgroups, associated with a CH reverse primer, or a mixture thereof, capable of specifically hybridizing in stringent conditions with the nucleic acid encoding the constant segment (CH) of a given type of an immunoglobulin heavy chain, **characterized in that** the set of VH forward primers comprises at least the 8 following subgroups of VH primers corresponding to the VH subgroups :

- the VH1 primers having the sequences SEQ ID N° 1 to SEQ ID N° 3, and
- the VH2 primer having the sequence SEQ ID N° 4, and
- the VH3a primers having the sequences SEQ ID N° 5 and SEQ ID N° 6, and
- the VH3b primers having the sequences SEQ ID N° 7 to SEQ ID N° 10, and
- the VH4 primers having the sequences SEQ ID N° 11 and SEQ ID N° 12, and
- the VH5 primer having the sequence SEQ ID N° 13, and
- the VH6 primer having the sequence SEQ ID N° 14, and
- the VH7 primer having the sequence SEQ ID N° 15.

**24.** The set of VH forward primers according to claim 23, **characterized in that** the sequences SEQ ID N° 1 to SEQ ID N° 15 may contain at least one to three point mutations, except for the nucleotides 1 to 6 of their 3' part.

**25.** The set of VH forward primers according to claim 23 or 24, **characterized in that** the CH reverse primer is selected from the CH reverse primers capable of specifically hybridizing in stringent conditions with the nucleic acids encoding the constant segments (CH) of the IgM heavy chain, the IgE heavy chain and the IgA heavy chain.

**26.** The set of VH forward primers according to claim 25, **characterized in that**, when the CH reverse primer is capable of specifically hybridizing in stringent conditions with the nucleic acid encoding the constant segment (CH) of the IgM heavy chain, the CH reverse primer has the sequence SEQ ID N° 26, or the sequence SEQ ID N° 26 wherein one to three point mutations may occur, except for the nucleotides 1 to 6 of its 3' part.

**27.** The set of VH forward primers according to claim 25, **characterized in that**, when the CH reverse primer is capable of specifically hybridizing in stringent conditions with the nucleic acid encoding the constant segment (CH) of the IgE heavy chain, the CH reverse primer has the sequence SEQ ID N° 33, or the sequence SEQ ID N° 33 wherein one to three point mutations may occur, except for the nucleotides 1 to 6 of its 3' part.

**28.** The set of VH forward primers according to claim 23 or 24, **characterized in that**, when the given type of immunoglobulin heavy chain is the IgG type, a mixture of two CH reverse primers is associated with the set of VH forward primers,
said two CH reverse primers having the sequences SEQ ID N° 27 and SEQ ID N° 28, or the sequences SEQ ID N° 27 and SEQ ID N° 28 wherein one to three point mutations may occur, except for the nucleotides 1 to 6 of its 3' part.

**29.** A method for the *in vitro* diagnosis of a condition selected from the group consisting of an auto-immune disease, a B cell lymphoma, an immunodepressive disease, and a condition which results from a bone marrow transplantation, from a vaccinal test or from an allergic reaction in a subject, **characterized in that** it comprises the following steps:

(1) determining the quantitative and qualitative profile of the repertoire of a given type of immunoglobulin heavy chain from a tissue sample of said subject, wherein said said determination comprises:

(a) obtaining either the cDNA from the mRNA expressed from the tissue sample or the cellular DNA extract of the tissue sample,
(b) performing the amplification of the cDNA obtained at the step (a) with a set of VH forward primers capable of specifically hybridizing in stringent conditions with the nucleic acids encoding the variable segments (VH) of immunoglobulin heavy chains, said variable segments being distributed among VH subgroups, associated with a CH reverse primer, or a mixture thereof, capable of specifically hybridizing in stringent conditions with the nucleic acid encoding the constant segment (CH) of a given type of an immunoglobulin heavy chain, and
(c) determining the quantitative and qualitative profile of the repertoire of said type of immunoglobulin heavy chain for each VH subgroup,

wherein the set of VH forward primers comprises at least the 8 following subgroups of VH primers corresponding to the VH subgroups :

- the VH1 primers having the sequences SEQ ID N° 1 to SEQ ID N° 3, and
- the VH2 primer having the sequence SEQ ID N° 4, and
- the VH3a primers having the sequences SEQ ID N° 5 and SEQ ID N° 6, and
- the VH3b primers having the sequences SEQ ID N° 7 to SEQ ID N° 10, and
- the VH4 primers having the sequences SEQ ID N° 11 and SEQ ID N° 12, and
- the VH5 primer having the sequence SEQ ID N° 13, and
- the VH6 primer having the sequence SEQ ID N° 14, and
- the VH7 primer having the sequence SEQ ID N° 15,

(2) comparing the quantitative and qualitative profile obtained at the step (1) with a control quantitative and qualitative profile of said given type of immunoglobulin heavy chain, the demonstration of a significant modification of the profile obtained at the step (1) being significant of such a condition in the subject.

**30.** A method for the *in vitro* follow-up of a treatment of a condition selected from the group consisting of an auto-immune disease, a B cell lymphoma, an immunodepressive disease, and a condition wich results from a bone marrow transplantation, from a vaccinal test or from an allergic reaction in a subject, **characterized in that** it comprises the following steps:

(1) optionally, determining before the treatment the quantitative and qualitative profile of the repertoire of a given type of immunoglobulin heavy chain from a tissue sample of said subject,
(2) determining, during the treatment, the quantitative and qualitative profile of the repertoire of a given type of immunoglobulin heavy chain at given times from tissue samples of said subject,

wherein said determination comprises:

(a) obtaining either the cDNA from the mRNA expressed from the tissue sample or the cellular DNA extract of the tissue sample,
(b) performing the amplification of the cDNA obtained at the step (a) with a set of VH forward primers capable of specifically hybridizing in stringent conditions with the nucleic acids encoding the variable segments (VH) of immunoglobulin heavy chains, said variable segments being distributed among VH subgroups, associated with a CH reverse primer, or a mixture thereof, capable of specifically hybridizing in stringent conditions with the nucleic acid encoding the constant segment (CH) of a given type of an immunoglobulin heavy chain, and
(c) determining the quantitative and qualitative profile of the repertoire of said type of immunoglobulin heavy chain for each VH subgroup,

wherein the set of VH forward primers comprises at least the 8 following subgroups of VH primers corresponding to the VH subgroups :

- the VH1 primers having the sequences SEQ ID N° 1 to SEQ ID N° 3, and
- the VH2 primer having the sequence SEQ ID N° 4, and
- the VH3a primers having the sequences SEQ ID N° 5 and SEQ ID N° 6, and
- the VH3b primers having the sequences SEQ ID N° 7 to SEQ ID N° 10, and
- the VH4 primers having the sequences SEQ ID N° 11 and SEQ ID N° 12, and
- the VH5 primer having the sequence SEQ ID N° 13, and
- the VH6 primer having the sequence SEQ ID N° 14, and
- the VH7 primer having the sequence SEQ ID N° 15,

(3) comparing the quantitative and qualitative profiles obtained at the step (2) and optionally at the step (1) with each others and optionally with a control quantitative and qualitative profile of the given type of immunoglobulin heavy chain, the demonstration of a significant modification of the profile obtained at the step (1) being significant of such a condition in the subject.

**31.** A kit for determining the quantitative and qualitative profile of the repertoire a given type of an immunoglobulin heavy chain expressed by a lymphocyte B population present in a tissue sample, **characterized in that** it comprises the set of VH forward primers according to anyone of claims 23 to 28 associated with the CH reverse primer.

**32.** The kit according to claim 31, **characterized in that** it further comprises a set of JH reverse primers, optionally labeled, corresponding to the JH subgroups and capable of specifically hybridizing in stringent conditions with the nucleic acids encoding the junction segments of the given type of immunoglobulin heavy chain.

**33.** The kit according to claim 32, **characterized in that** the set of JH reverse primers comprises the 6 following subgroups of JH primers corresponding to the JH subgroups :

- the JH1 primer having the sequence SEQ ID N° 16, and
- the JH2 primer having the sequence SEQ ID N° 17, and
- the JH3 primer having the sequence SEQ ID N° 18, and
- the JH4 primers having the sequences SEQ ID N° 19 to SEQ ID N° 21, and
- the JH5 primer having the sequence SEQ ID N° 22, and
- the JH6 primers having the sequences SEQ ID N° 23 to SEQ ID N° 25.

**34.** The kit according to claim 33, **characterized in that** the sequences SEQ ID N° 16 to SEQ ID N° 25 may contain at least one to three point mutations, except for the nucleotides 1 to 6 of their 3' part.

**35.** Use of the kit according to anyone of claims 31 to 34, for the *in vitro* diagnosis of a condition selected from the group consisting of an auto-immune diseasse, a B cell lymphoma, an immunodepressive disease, and a condition which results from a bone marrow transplantation, from a vaccinal test or from an allergic reaction in a subjet.

## Patentansprüche

1. Verfahren zum Bestimmen des quantitativen und qualitativen Profils des Repertoires eines gegebenen Typs einer schweren Immunglobulinkette, die durch eine in einer Gewebeprobe vorhandene B-Lymphozyten-Population exprimiert wird, **dadurch gekennzeichnet, dass** es die folgenden Schritte umfasst:

   (a) Gewinnen entweder der cDNA ausgehend von der mRNA, die ausgehend von der Gewebeprobe exprimiert wird, oder des zellulären DNA-Extrakts der Gewebeprobe,
   (b) Ausführen der Amplifizierung der in dem Schritt (a) erhaltenen cDNA mit einem Satz von VH-Vorwärts-Primern, der in der Lage ist, unter stringenten Bedingungen spezifisch mit den Nukleinsäuren, die die variablen Segmente (VH) von schweren Immunglobulinketten kodieren, zu hybridisieren, wobei die variablen Segmente unter VH-Untergruppen verteilt sind, kombiniert mit einem CH-Reverse-Primer oder einer Mischung davon, der bzw. die in der Lage ist, unter stringenten Bedingungen spezifisch mit der Nukleinsäure, die das konstante Segment (CH) eines gegebenen Typs einer schweren Immunglobulinkette kodiert, zu hybridisieren, und
   (c) Bestimmen des quantitativen und qualitativen Profils des Repertoires von dem Typ einer schweren Immunglobulinkette für jede VH-Untergruppe,

   **dadurch gekennzeichnet, dass** der Satz von VH-Vorwärts-Primern mindestens die 8 folgenden Untergruppen von VH-Primern, welche den VH-Untergruppen entsprechen, umfasst:

   - die VH1-Primer mit den Sequenzen SEQ ID NO: 1 bis SEQ ID NO: 3 und
   - den VH2-Primer mit der Sequenz SEQ ID NO: 4 und
   - die VH3a-Primer mit den Sequenzen SEQ ID NO: 5 und SEQ ID NO: 6 und
   - die VH3b-Primer mit den Sequenzen SEQ ID NO: 7 bis SEQ ID NO: 10 und
   - die VH4-Primer mit den Sequenzen SEQ ID NO: 11 und SEQ ID NO: 12 und
   - den VH5-Primer mit der Sequenz SEQ ID NO: 13 und
   - den VH6-Primer mit der Sequenz SEQ ID NO: 14 und
   - den VH7-Primer mit der Sequenz SEQ ID NO: 15.

2. Verfahren zum Bestimmen des quantitativen und qualitativen Profils nach Anspruch 1, **dadurch gekennzeichnet, dass** getrennte Amplifizierungen für jede der VH-Untergruppen ausgeführt werden.

3. Verfahren zum Bestimmen des quantitativen und qualitativen Profils nach Anspruch 2, **dadurch gekennzeichnet, dass** die getrennten Amplifizierungen getrennte Echtzeit-Amplifizierungen sind, wobei die Echtzeit-Amplifizierungen ausgeführt werden unter Verwendung einer markierten CH-Reverse-Sonde, vorzugsweise einer markierten CH-Reverse-Hydrolyse-Sonde, die in der Lage ist, unter stringenten Bedingungen spezifisch mit dem konstanten Segment des gegebenen Typs einer schweren Immunglobulinkette zu hybridisieren, und in der Lage ist, ein nachweisbares Signal zu emittieren jedes Mal, wenn jeder Amplifizierungszyklus stattfindet, und **dadurch gekennzeichnet, dass** das für jede VH-Untergruppe erhaltene Signal gemessen wird.

4. Verfahren zum Bestimmen des quantitativen und qualitativen Profils nach Anspruch 2 oder 3, **dadurch gekennzeichnet, dass** die für jede der VH-Untergruppen erhaltenen getrennten Amplifizierungsprodukte weiter verlängert werden unter Verwendung einer markierten CH-Reverse-Sonde, die in der Lage ist, unter stringenten Bedingungen spezifisch mit dem konstanten Segment des gegebenen Typs einer schweren Immunglobulinkette zu hybridisieren, und in der Lage ist, ein nachweisbares Signal zu emittieren, und **dadurch gekennzeichnet, dass** die Verlängerungsprodukte für jede der VH-Untergruppen bezogen auf ihre Länge aufgetrennt werden, das für die aufgetrennten Verlängerungsprodukte erhaltene Signal gemessen wird und das quantitative und qualitative Profil der Markierungsintensität bezogen auf die Verlängerungsproduktlänge für jede der VH-Untergruppen individuell ermittelt wird.

5. Verfahren zum Bestimmen des quantitativen und qualitativen Profils nach einem der Ansprüche 1 bis 4, **dadurch gekennzeichnet, dass** die Sequenzen SEQ ID NO: 1 bis SEQ ID NO: 15 mindestens eine bis drei Punktmutationen mit Ausnahme der Nukleotide 1 bis 6 von deren 3'-Abschnitt enthalten können.

6. Verfahren zum Bestimmen des quantitativen und qualitativen Profils nach Anspruch 1 bis 5, **dadurch gekennzeichnet, dass** der CH-Reverse-Primer aus den CH-Reverse-Primern, die in der Lage sind, unter stringenten Bedingungen spezifisch mit den Nukleinsäuren, die die konstanten Segmente (CH) der schweren Kette von IgM, der schweren Kette von IgE und der schweren Kette von IgA kodieren, zu hybridisieren, ausgewählt ist.

7. Verfahren zum Bestimmen des quantitativen und qualitativen Profils nach Anspruch 6, **dadurch gekennzeichnet, dass**, wenn der CH-Reverse-Primer in der Lage ist, unter stringenten Bedingungen spezifisch mit der Nukleinsäure, die das konstante Segment (CH) der schweren Kette von IgM kodiert, zu hybridisieren, der CH-Reverse-Primer die Sequenz SEQ ID NO: 26 oder die Sequenz SEQ ID NO: 26, in welcher eine bis drei Punktmutationen mit Ausnahme der Nukleotide 1 bis 6 von deren 3'-Abschnitt auftreten können, aufweist.

8. Verfahren zum Bestimmen des quantitativen und qualitativen Profils nach Anspruch 6, **dadurch gekennzeichnet, dass**, wenn der CH-Reverse-Primer in der Lage ist, unter stringenten Bedingungen spezifisch mit der Nukleinsäure, die das konstante Segment (CH) der schweren Kette von IgE kodiert, zu hybridisieren, der CH-Reverse-Primer die Sequenz SEQ ID NO: 33 oder die Sequenz SEQ ID NO: 33, in welcher eine bis drei Punktmutationen mit Ausnahme der Nukleotide 1 bis 6 von deren 3'-Abschnitt auftreten können, aufweist.

9. Verfahren zum Bestimmen des quantitativen und qualitativen Profils nach einem der Ansprüche 1 bis 5, **dadurch gekennzeichnet, dass**, wenn der gegebene Typ einer schweren Immunglobulinkette der IgG-Typ ist, eine Mischung von zwei CH-Reverse-Primern mit dem Satz von VH-Vorwärts-Primern kombiniert wird,
wobei die beiden CH-Reverse-Primer die Sequenzen SEQ ID NO: 27 und SEQ ID NO: 28 oder die Sequenzen SEQ ID NO: 27 und SEQ ID NO: 28, in welchen eine bis drei Punktmutationen mit Ausnahme der Nukleotide 1 bis 6 von deren 3'-Abschnitt auftreten können, aufweisen.

10. Verfahren zum Bestimmen des quantitativen und qualitativen Profils nach Anspruch 6 oder 7, **dadurch gekennzeichnet, dass**, wenn der gegebene Typ einer schweren Immunglobulinkette eine schwere Kette eines IgM ist und wenn die getrennten Amplifizierungen getrennte Echtzeit-Amplifizierungen sind, die markierte CH-Hydrolyse-Sonde die Sequenz SEQ ID NO: 29 oder die Sequenz SEQ ID NO: 29, in welcher eine oder zwei Punktmutationen auftreten können, aufweist.

11. Verfahren zum Bestimmen des quantitativen und qualitativen Profils nach Anspruch 6 oder 7, **dadurch gekennzeichnet, dass**, wenn der gegebene Typ einer schweren Immunglobulinkette eine schwere Kette eines IgM ist und wenn die getrennten Amplifizierungsprodukte, die für jede der VH-Untergruppen erhalten werden, weiter verlängert werden, die markierte CH-Reverse-Sonde die Sequenz SEQ ID NO: 30 oder die Sequenz SEQ ID NO: 30, in welcher eine bis drei Punktmutationen mit Ausnahme der Nukleotide 1 bis 6 von deren 3'-Abschnitt auftreten können, aufweist.

12. Verfahren zum Bestimmen des quantitativen und qualitativen Profils nach Anspruch 6 oder 8, **dadurch gekennzeichnet, dass**, wenn der gegebene Typ einer schweren Immunglobulinkette eine schwere Kette eines IgE ist und wenn die getrennten Amplifizierungen getrennte Echtzeit-Amplifizierungen sind, die markierte CH-Hydrolyse-Sonde die Sequenz SEQ ID NO: 36 oder die Sequenz SEQ ID NO: 36, in welcher eine oder zwei Punktmutationen auftreten können, aufweist.

13. Verfahren zum Bestimmen des quantitativen und qualitativen Profils nach Anspruch 6 oder 8, **dadurch gekennzeichnet, dass**, wenn der gegebene Typ einer schweren Immunglobulinkette eine schwere Kette eines IgE ist und wenn die getrennten Amplifizierungsprodukte, die für jede der VH-Untergruppen erhalten werden, weiter verlängert werden, die markierte CH-Reverse-Sonde die Sequenz SEQ ID NO: 37 oder die Sequenz SEQ ID NO: 37, in welcher eine bis drei Punktmutationen mit Ausnahme der Nukleotide 1 bis 6 von deren 3'-Abschnitt auftreten können, aufweist.

14. Verfahren zum Bestimmen des quantitativen und qualitativen Profils nach Anspruch 9, **dadurch gekennzeichnet, dass**, wenn der gegebene Typ einer schweren Immunglobulinkette eine schwere Kette eines IgG ist und wenn die getrennten Amplifizierungen getrennte Echtzeit-Amplifizierungen sind, die markierte CH-Hydrolyse-Sonde die Sequenz SEQ ID NO: 34 oder die Sequenz SEQ ID NO: 34, in welcher eine oder zwei Punktmutationen auftreten können, aufweist.

15. Verfahren zum Bestimmen des quantitativen und qualitativen Profils nach Anspruch 9, **dadurch gekennzeichnet, dass**, wenn der gegebene Typ einer schweren Immunglobulinkette eine schwere Kette eines IgG ist und wenn die getrennten Amplifizierungsprodukte, die für jede der VH-Untergruppen erhalten werden, weiter verlängert werden, die markierte CH-Reverse-Sonde die Sequenz SEQ ID NO: 35 oder die Sequenz SEQ ID NO: 35, in welcher eine bis drei Punktmutationen mit Ausnahme der Nukleotide 1 bis 6 von deren 3'-Abschnitt auftreten können, aufweist.

16. Verfahren zum Bestimmen des quantitativen und qualitativen Profils nach einem der Ansprüche 2 bis 15, **dadurch gekennzeichnet, dass** weitere getrennte Amplifizierungen für jede der JH-Untergruppen ausgehend von den getrennten Amplifizierungsprodukten, die für mindestens eine gegebene VH-Untergruppe der VH-Untergruppen mit dem CH-Reverse-Primer erhalten werden, ausgeführt werden,
wobei die weiteren getrennten Amplifizierungen ausgeführt werden unter Verwendung eines internen VH-Vorwärts-Primers, welcher der gegebenen VH-Untergruppe entspricht, und kombiniert mit einem Satz von JH-Reverse-Primern, welche den JH-Untergruppen entsprechen und in der Lage sind, unter stringenten Bedingungen spezifisch mit den Nukleinsäuren, die die Verbindungssegmente ("Junction"-Segmente) des gegebenen Typs einer schweren Immunglobulinkette kodieren, zu hybridisieren.

**17.** Verfahren zum Bestimmen des quantitativen und qualitativen Profils nach Anspruch 16, **dadurch gekennzeichnet, dass** die weiteren getrennten Amplifizierungen Echtzeit-Amplifizierungen sind, die unter Verwendung einer markierten VH-Vorwärts-Sonde, vorzugsweise einer markierten VH-Vorwärts-Hydrolyse-Sonde, die in der Lage ist, unter stringenten Bedingungen spezifisch mit dem variablen Segment des gegebenen Typs einer schweren Immunglobulinkette zu hybridisieren, und in der Lage ist, ein nachweisbares Signal zu emittieren jedes Mal, wenn jeder Amplifizierungszyklus stattfindet, ausgeführt werden, und **dadurch gekennzeichnet, dass** das für jede JH-Untergruppe erhaltene Signal gemessen wird.

**18.** Verfahren zum Bestimmen des quantitativen und qualitativen Profils nach den Ansprüchen 16 oder 17, **dadurch gekennzeichnet, dass**, wenn die gegebene VH-Untergruppe die VH5-Untergruppe ist, der interne VH5-Vorwärts-Primer die Sequenz SEQ ID NO: 31 oder die Sequenz SEQ ID NO: 31, in welcher eine bis drei Punktmutationen mit Ausnahme der Nukleotide 1 bis 6 von deren 3'-Abschnitt auftreten können, aufweist.

**19.** Verfahren zum Bestimmen des quantitativen und qualitativen Profils nach Anspruch 18, **dadurch gekennzeichnet, dass** die markierte VH-Vorwärts-Hydrolyse-Sonde die Sequenz SEQ ID NO: 32 oder die Sequenz SEQ ID NO: 32, in welcher eine oder zwei Punktmutationen auftreten können, aufweist.

**20.** Verfahren zum Bestimmen des quantitativen und qualitativen Profils nach einem der Ansprüche 2 bis 15, **dadurch gekennzeichnet, dass** getrennte Verlängerungen für jede der JH-Untergruppen ausgehend von den getrennten Amplifizierungsprodukten, die für mindestens eine gegebene VH-Untergruppe von den VH-Untergruppen mit dem CH-Reverse-Primer erhalten werden, ausgeführt werden,
wobei die weiteren getrennten Verlängerungen ausgeführt werden unter Verwendung eines Satzes von markierten JH-Reverse-Primern, welche JH-Untergruppen entsprechen und in der Lage sind, unter stringenten Bedingungen spezifisch mit den Nukleinsäuren, die die Verbindungssegmente ("Junction"-Segmente) des gegebenen Typs einer schweren Immunglobulinkette kodieren, zu hybridisieren, wobei die markierten JH-Reverse-Primer in der Lage sind, ein nachweisbares Signal zu emittieren,
und **dadurch gekennzeichnet, dass** die Verlängerungsprodukte für jede der JH-Untergruppen bezogen auf ihre Länge aufgetrennt werden, das für die aufgetrennten Verlängerungsprodukte erhaltene Signal gemessen wird und das quantitative und qualitative Profil der Markierungsintensität bezogen auf die Verlängerungsproduktlänge für jede der JH-Untergruppen für die gegebene VH-Untergruppe ermittelt wird.

**21.** Verfahren zum Bestimmen des quantitativen und qualitativen Profils nach einem der Ansprüche 16 bis 20, **dadurch gekennzeichnet, dass** der Satz von JH-Vorwärts-Primern, die gegebenenfalls markiert sind, mindestens die 6 folgenden Untergruppen von JH-Primern, welche den JH-Untergruppen entsprechen, umfasst:

- den JH1-Primer mit der Sequenz SEQ ID NO: 16 und
- den JH2-Primer mit der Sequenz SEQ ID NO: 17 und
- den JH3-Primer mit der Sequenz SEQ ID NO: 18 und
- die JH4-Primer mit den Sequenzen SEQ ID NO: 19 bis SEQ ID NO: 21 und
- den JH5-Primer mit der Sequenz SEQ ID NO: 22 und
- die JH6-Primer mit den Sequenzen SEQ ID NO: 23 bis SEQ ID NO: 25.

**22.** Verfahren zum Bestimmen des quantitativen und qualitativen Profils nach Anspruch 21, **dadurch gekennzeichnet, dass** die Sequenzen SEQ ID NO: 16 bis SEQ ID NO: 25 mindestens eine bis drei Punktmutationen mit Ausnahme der Nukleotide 1 bis 6 von deren 3'-Abschnitt enthalten können.

**23.** Satz von VH-Vorwärts-Primern, die in der Lage sind, unter stringenten Bedingungen spezifisch mit den Nukleinsäuren, die die variablen Segmente (VH) von schweren Immunglobulinkette kodieren, zu hybridisieren, wobei die variablen Segmente unter mindestens 8 VH-Untergruppen verteilt sind, kombiniert mit einem CH-Reverse-Primer oder einer Mischung davon, der bzw. die in der Lage ist, unter stringenten Bedingungen spezifisch mit der Nukleinsäure, die das konstante Segment (CH) eines gegebenen Typs einer schweren Immunglobulinkette kodiert, zu hybridisieren, **dadurch gekennzeichnet, dass** der Satz von VH-Vorwärts-Primern mindestens die 8 folgenden Untergruppen von VH-Primern, welche den VH-Untergruppen entsprechen, umfasst:

- die VH1-Primer mit den Sequenzen SEQ ID NO: 1 bis SEQ ID NO: 3 und
- den VH2-Primer mit der Sequenz SEQ ID NO: 4 und
- die VH3a-Primer mit den Sequenzen SEQ ID NO: 5 und SEQ ID NO: 6 und
- die VH3b-Primer mit den Sequenzen SEQ ID NO: 7 bis SEQ ID NO: 10 und
- die VH4-Primer mit den Sequenzen SEQ ID NO: 11 und SEQ ID NO: 12 und
- den VH5-Primer mit der Sequenz SEQ ID NO: 13 und
- den VH6-Primer mit der Sequenz SEQ ID NO: 14 und
- den VH7-Primer mit der Sequenz SEQ ID NO: 15.

**24.** Satz von VH-Vorwärts-Primern nach Anspruch 23, **dadurch gekennzeichnet, dass** die Sequenzen SEQ ID NO: 1 bis SEQ ID NO: 15 mindestens eine bis drei Punktmutationen mit Ausnahme der Nukleotide 1 bis 6 von deren 3'-Abschnitt enthalten können.

**25.** Satz von VH-Vorwärts-Primern nach Anspruch 23 oder 24, **dadurch gekennzeichnet, dass** der CH-Reverse-Primer aus den CH-Reverse-Primern, die in der Lage sind, unter stringenten Bedingungen spezifisch mit den Nukleinsäuren, die die konstanten Segmente (CH) der schweren Kette von IgM, der schweren Kette von IgE und der schweren Kette von IgA kodieren, zu hybridisieren, ausgewählt ist.

**26.** Satz von VH-Vorwärts-Primern nach Anspruch 25, **dadurch gekennzeichnet, dass**, wenn der CH-Reverse-Primer in der Lage ist, unter stringenten Bedingungen spezifisch mit der Nukleinsäure, die das konstante Segment (CH) der schweren Kette von IgM kodiert, zu hybridisieren, der CH-Reverse-Primer die Sequenz SEQ ID NO: 26 oder die Sequenz SEQ ID NO: 26, in welcher eine bis drei Punktmutationen mit Ausnahme der Nukleotide 1 bis 6 von deren 3'-Abschnitt auftreten können, aufweist.

**27.** Satz von VH-Vorwärts-Primern nach Anspruch 25, **dadurch gekennzeichnet, dass**, wenn der CH-Reverse-Primer in der Lage ist, unter stringenten Bedingungen spezifisch mit der Nukleinsäure, die das konstante Segment (CH) der schweren Kette von IgE kodiert, zu hybridisieren, der CH-Reverse-Primer die Sequenz SEQ ID NO: 33 oder die Sequenz SEQ ID NO: 33, in welcher eine bis drei Punktmutationen mit Ausnahme der Nukleotide 1 bis 6 von deren 3'-Abschnitt auftreten können, aufweist.

**28.** Satz von VH-Vorwärts-Primern nach Anspruch 23 oder 24, **dadurch gekennzeichnet, dass**, wenn der gegebene Typ einer schweren Immunglobulinkette der IgG-Typ ist, eine Mischung von zwei CH-Reverse-Primern mit dem Satz von VH-Vorwärts-Primern kombiniert wird,
wobei die zwei CH-Reverse-Primer die Sequenzen SEQ ID NO: 27 und SEQ ID NO: 28 oder die Sequenzen SEQ ID NO: 27 und SEQ ID NO: 28, in welchen eine bis drei Punktmutationen mit Ausnahme der Nukleotide 1 bis 6 von deren 3'-Abschnitt auftreten können, aufweisen.

**29.** Verfahren zur *in vitro*-Diagnose eines Zustands, ausgewählt aus der Gruppe bestehend aus einer Autoimmunerkrankung, einem B-Zell-Lymphom, einer immunsuppressiven Erkrankung und einem Zustand, der aus einer Knochenmarkstransplantation, aus einem Impftest oder aus einer allergischen Reaktion in einem Individuum resultiert, **dadurch gekennzeichnet, dass** es die folgenden Schritte umfasst:

(1) Bestimmen des quantitativen und qualitativen Profils des Repertoires eines gegebenen Typs einer schweren Immunglobulinkette ausgehend von einer Gewebeprobe des Individuums, wobei die Bestimmung umfasst:

(a) Gewinnen entweder der cDNA ausgehend von der mRNA, die ausgehend von der Gewebeprobe exprimiert wird, oder des zellulären DNA-Extrakts der Gewebeprobe,
(b) Ausführen der Amplifizierung der in dem Schritt (a) erhaltenen cDNA mit einem Satz von VH-Vorwärts-Primern, der in der Lage ist, unter stringenten Bedingungen spezifisch mit den Nukleinsäuren, die die variablen Segmente (VH) von schweren Immunglobulinketten kodieren, zu hybridisieren, wobei die variablen Segmente unter VH-Untergruppen verteilt sind, kombiniert mit einem CH=Reverse-Primer oder einer Mischung davon, der bzw. die in der Lage ist, unter stringenten Bedingungen spezifisch mit der Nukleinsäure, die das konstante Segment (CH) eines gegebenen Typs einer schweren Immunglobulinkette kodiert, zu hybridisieren, und
(c) Bestimmen des quantitativen und qualitativen Profils des Repertoires von dem Typ einer schweren Immunglobulinkette für jede VH-Untergruppe,

wobei der Satz von VH-Vorwärts-Primern mindestens die 8 folgenden Untergruppen von VH-Primern, welche den VH-Untergruppen entsprechen, umfasst:

- die VH1-Primer mit den Sequenzen SEQ ID NO: 1 bis SEQ ID NO: 3 und
- den VH2-Primer mit der Sequenz SEQ ID NO: 4 und
- die VH3a-Primer mit den Sequenzen SEQ ID NO: 5 und SEQ ID NO: 6 und
- die VH3b-Primer mit den Sequenzen SEQ ID NO: 7 bis SEQ ID NO: 10 und
- die VH4-Primer mit den Sequenzen SEQ ID NO: 11 und SEQ ID NO: 12 und
- den VH5-Primer mit der Sequenz SEQ ID NO: 13 und
- den VH6-Primer mit der Sequenz SEQ ID NO: 14 und
- den VH7-Primer mit der Sequenz SEQ ID NO: 15,

(2) Vergleichen des in dem Schritt (1) erhaltenen quantitativen und qualitativen Profils mit einem quantitativen und qualitativen Kontrollprofil von dem gleichen Typ einer schweren Immunglobulinkette, wobei das Aufzeigen einer signifikanten Modifizierung des in dem Schritt (1) erhaltenen Profils für einen solchen Zustand in dem Individuum signifikant ist.

**30.** Verfahren zur *in vitro*-Nachbetreuung einer Behandlung eines Zustands, ausgewählt aus der Gruppe bestehend aus einer Autoimmunerkrankung, einem B-Zell-Lymphom, einer immunsuppressiven Erkrankung und einem Zustand, der aus einer Knochenmarkstransplantation, aus einem Impftest oder aus einer allergischen Reaktion in einem Individuum resultiert, **dadurch gekennzeichnet, dass** es die folgenden Schritte umfasst:

(1) gegebenenfalls Bestimmen des quantitativen und qualitativen Profils des Repertoires eines gegebenen Typs einer schweren Immunglobulinkette ausgehend von einer Gewebeprobe des Individuums vor der Behandlung,
(2) Bestimmen des quantitativen und qualitativen Profils des Repertoires eines gegebenen Typs einer schweren Immunglobulinkette ausgehend von Gewebeproben des Individuums während der Behandlung zu gegebenen Zeitpunkten,
wobei die Bestimmung umfasst:

(a) Gewinnen entweder der cDNA ausgehend von der mRNA, die ausgehend von der Gewebeprobe exprimiert wird, oder des zellulären DNA-Extrakts der Gewebeprobe,
(b) Ausführen der Amplifizierung der in dem Schritt (a) erhaltenen cDNA mit einem Satz von VH-Vorwärts-Primern, der in der Lage ist, unter stringenten Bedingungen spezifisch mit den Nukleinsäuren, die die variablen Segmente (VH) von schweren Immunglobulinketten kodieren, zu hybridisieren, wobei die variablen Segmente unter VH-Untergruppen verteilt sind, kombiniert mit einem CH-Reverse-Primer oder einer Mischung davon, der bzw. die in der Lage ist, unter stringenten Bedingungen spezifisch mit der Nukleinsäure, die das konstante Segment (CH) eines gegebenen Typs einer schweren Immunglobulinkette kodiert, zu hybridisieren, und
(c) Bestimmen des quantitativen und qualitativen Profils des Repertoires von dem Typ einer schweren Immunglobulinkette für jede VH-Untergruppe,

wobei der Satz von VH-Vorwärts-Primern mindestens die 8 folgenden Untergruppen von VH-Primern, welche den VH-Untergruppen entsprechen, umfasst:

- die VH1-Primer mit den Sequenzen SEQ ID NO: 1 bis SEQ ID NO: 3 und
- den VH2-Primer mit der Sequenz SEQ ID NO: 4 und
- die VH3a-Primer mit den Sequenzen SEQ ID NO: 5 und SEQ ID NO: 6 und
- die VH3b-Primer mit den Sequenzen SEQ ID NO: 7 bis SEQ ID NO: 10 und
- die VH4-Primer mit den Sequenzen SEQ ID NO: 11 und SEQ ID NO: 12 und
- den VH5-Primer mit der Sequenz SEQ ID NO: 13 und
- den VH6-Primer mit der Sequenz SEQ ID NO: 14 und
- den VH7-Primer mit der Sequenz SEQ ID NO: 15,

(3) Vergleichen der in dem Schritt (2) und gegebenenfalls in dem Schritt (1) erhaltenen quantitativen und qualitativen Profile mit einander und gegebenenfalls mit einem quantitativen und qualitativen Kontrollprofil von dem gegebenen Typ einer schweren Immunglobulinkette, wobei das Aufzeigen einer signifikanten Modifizierung des in dem Schritt (1) erhaltenen Profils für einen solchen Zustand in dem Individuum signifikant ist.

**31.** Kit zum Bestimmen des quantitativen und qualitativen Profils des Repertoires eines gegebenen Typs einer schweren Immunglobulinkette, die durch eine in einer Gewebeprobe vorhandene B-Lymphozyten-Population exprimiert wird, **dadurch gekennzeichnet, dass** er den Satz von VH-Vorwärts-Primern nach einem der Ansprüche 23 bis 28 kombiniert mit dem CH-Reverse-Primer umfasst.

**32.** Kit nach Anspruch 31, **dadurch gekennzeichnet, dass** er des Weiteren einen Satz von gegebenenfalls markierten JH-Reverse-Primern, welche den JH-Untergruppen entsprechen und in der Lage sind, unter stringenten Bedingungen spezifisch mit den Nukleinsäuren, die die Verbindungssegmente ("Junction"-Segmente) des gegebenen Typs einer schweren Immunglobulinkette kodieren, zu hybridisieren, umfasst.

**33.** Kit nach Anspruch 32, **dadurch gekennzeichnet, dass** der Satz von JH-Reverse-Primern die 6 folgenden Untergruppen von JH-Primern, welche den JH-Untergruppen entsprechen, umfasst:

- den JH1-Primer mit der Sequenz SEQ ID NO: 16 und
- den JH2-Primer mit der Sequenz SEQ ID NO: 17 und
- den JH3-Primer mit der Sequenz SEQ ID NO: 18 und
- die JH4-Primer mit den Sequenzen SEQ ID NO: 19 bis SEQ ID NO: 21 und
- den JH5-Primer mit der Sequenz SEQ ID NO: 22 und
- die JH6-Primer mit den Sequenzen SEQ ID NO: 23 bis SEQ ID NO: 25.

**34.** Kit nach Anspruch 33, **dadurch gekennzeichnet, dass** die Sequenzen SEQ ID NO: 16 bis SEQ ID NO: 25 mindestens eine bis drei Punktmutationen mit Ausnahme der Nukleotide 1 bis 6 von deren 3'-Abschnitt enthalten können.

**35.** Verwendung des Kits nach einem der Ansprüche 31 bis 34 für die *in vitro*-Diagnose eines Zustands, ausgewählt aus der Gruppe bestehend aus einer Autoimmunerkrankung, einem B-Zell-Lymphom, einer immunsuppressiven Erkrankung und einem Zustand, der aus einer Knochenmarkstransplantation, aus einem Impftest oder aus einer allergischen Reaktion in einem Individuum resultiert.

## Revendications

**1.** Procédé de détermination du profil quantitatif et qualitatif du répertoire d'un type donné d'une chaîne lourde d'immunoglobuline, exprimé par une population de lymphocytes B présents dans un échantillon de tissu, **caractérisé en ce qu'**il comprend les étapes suivantes consistant à :

(a) obtenir soit l'ADNc provenant de l'ARNm exprimé à partir de l'échantillon de tissu soit l'extrait d'ADN cellulaire de l'échantillon de tissu,
(b) réaliser l'amplification de l'ADNc obtenu à l'étape (a) avec un ensemble d'amorces sens VH susceptibles d'hybridation spécifique dans des conditions stringentes avec les acides nucléiques codant les segments variables (VH) des chaînes lourdes d'immunoglobuline, lesdits segments variables étant distribués parmi des sous-groupes VH, associés à une amorce antisens CH, ou un mélange de ceux-ci, susceptibles d'hybridation spécifique, dans des conditions stringentes, avec l'acide nucléique codant le segment constant (CH) d'un type donné d'une chaîne lourde d'immunoglobuline, et
(c) déterminer le profil quantitatif et qualitatif du répertoire dudit type de chaîne lourde d'immunoglobuline pour chaque sous-groupe VH,

**caractérisé en ce que** l'ensemble des amorces sens VH comprend au moins les 8 sous-groupes suivants d'amorces VH correspondant aux sous-groupes VH :

- les amorces VH1 ayant les séquences SEQ ID N°1 à SEQ ID N°3, et
- l'amorce VH2 ayant la séquence SEQ ID N°4, et
- les amorces VH3a ayant les séquences SEQ ID N°5 et SEQ ID N°6, et
- les amorces VH3b ayant les séquences SEQ ID N° 7 à SEQ ID N°10, et
- les amorces VH4 ayant les séquences SEQ ID N°11 et SEQ ID N°12, et
- l'amorce VH5 ayant la séquence SEQ ID N°13, et
- l'amorce VH6 ayant la séquence SEQ ID N°14, et

- l'amorce VH7 ayant la séquence SEQ ID N°15.

2. Procédé de détermination du profil quantitatif et qualitatif selon la revendication 1, **caractérisé en ce que** des amplifications séparées sont réalisées pour chacun des sous-groupes VH.

3. Procédé de détermination du profil quantitatif et qualitatif selon la revendication 2, **caractérisé en ce que** les amplifications séparées sont des amplifications séparées en temps réel, lesdites amplifications en temps réel étant réalisées en utilisant une sonde antisens marquée CH, de préférence une sonde d'hydrolyse antisens marquée CH, susceptible d'hybridation spécifique dans des conditions stringentes avec le segment constant du type donné de chaîne lourde d'immunoglobuline et susceptible d'émettre un signal détectable chaque fois qu'un cycle d'amplification se produit, et **caractérisé en ce que** le signal obtenu pour chaque sous-groupe VH est mesuré.

4. Procédé de détermination du profil quantitatif et qualitatif selon la revendication 2 ou 3, **caractérisé en ce que** les produits d'amplifications séparées obtenus pour chacun des sous-groupes VH sont encore élongués en utilisant une sonde antisens marquée CH susceptible d'hybridation spécifique dans des conditions stringentes avec le segment constant du type donné de la chaîne lourde d'immunoglobuline et susceptible d'émettre un signal détectable, et
**caractérisé en ce que** les produits d'élongation sont séparés, pour chacun des sous-groupes VH, par rapport à leur longueur, le signal obtenu pour les produits d'élongation séparés est mesuré et le profil quantitatif et qualitatif de l'intensité de marquage par rapport à la longueur de produit d'élongation est établi, pour chacun des sous- groupes de VH individuellement.

5. Procédé de détermination du profil quantitatif et qualitatif selon l'une quelconque des revendications 1 à 4, **caractérisé en ce que** les séquences SEQ ID N°1 à SEQ ID N°15 peuvent contenir au moins une à trois mutations ponctuelles, sauf pour les nucléotides 1 à 6 de leur partie 3'.

6. Procédé de détermination du profil quantitatif et qualitatif selon la revendication 1 à 5, **caractérisé en ce que** l'amorce antisens CH est choisie parmi les amorces antisens CH susceptibles d'hybridation spécifique dans des conditions stringentes avec les acides nucléiques codant les segments constants (CH) de la chaîne lourde d'IgM, la chaîne lourde d'IgE et la chaîne lourde d'IgA.

7. Procédé de détermination du profil quantitatif et qualitatif selon la revendication 6, **caractérisé en ce que**, quand l'amorce antisens CH est susceptible d'hybridation spécifique dans des conditions stringentes avec l'acide nucléique codant le segment constant (CH) de la chaîne lourde d'IgM, l'amorce antisens CH a la séquence SEQ ID N°26, ou la séquence SEQ ID N°26 dans laquelle une à trois mutations ponctuelles peuvent exister, sauf pour les nucléotides 1 à 6 de sa partie 3'.

8. Procédé de détermination du profil quantitatif et qualitatif selon la revendication 6, **caractérisé en ce que**, quand l'amorce antisens CH est susceptible d'hybridation spécifique dans des conditions stringentes avec l'acide nucléique codant le segment constant (CH) de la chaîne lourde d'IgE, l'amorce antisens CH a la séquence SEQ ID N°33, ou la séquence SEQ ID N°33 dans laquelle une à trois mutations ponctuelles peuvent exister, sauf pour les nucléotides 1 à 6 de sa partie 3'.

9. Procédé de détermination du profil quantitatif et qualitatif selon l'une quelconque des revendications 1 à 5, **caractérisé en ce que**, quand le type donné de chaîne lourde d'immunoglobuline est le type IgG, un mélange des deux amorces antisens CH est associé à l'ensemble des amorces sens VH,
lesdites deux amorces antisens CH ayant les séquences SEQ ID N°27 et SEQ ID N°28, ou les séquences SEQ ID N°27 et SEQ ID N°28 dans lesquelles une à trois mutations ponctuelles peuvent exister, sauf pour les nucléotides 1 à 6 de sa partie 3'.

10. Procédé de détermination du profil quantitatif et qualitatif selon la revendication 6 ou 7, **caractérisé en ce que**, quand le type donné de la chaîne lourde d'immunoglobuline est une chaîne lourde d'IgM et quand les amplifications séparées sont des amplifications séparées en temps réel, la sonde d'hydrolyse marquée CH a la séquence SEQ ID N°29, ou la séquence SEQ ID N°29 dans laquelle une ou deux mutations ponctuelles peuvent exister.

11. Procédé de détermination du profil quantitatif et qualitatif selon la revendication 6 ou 7, **caractérisé en ce que**, quand le type donné de la chaîne lourde d'immunoglobuline est une chaîne lourde d'IgM et quand les produits d'amplifications séparées obtenus pour chacun des sous-groupes VH sont encore élongués, la sonde antisens marquée CH a la séquence SEQ ID N°30, ou la séquence SEQ ID N°30 dans laquelle une à trois mutations ponctuelles peuvent exister, sauf pour les nucléotides 1 à 6 de sa partie 3'.

**12.** Procédé de détermination du profil quantitatif et qualitatif selon la revendication 6 ou 8, **caractérisé en ce que**, quand le type donné de la chaîne lourde d'immunoglobuline est une chaîne lourde d'IgE et quand les amplifications séparées sont des amplifications séparées en temps réel, la sonde d'hydrolyse marquée CH a la séquence SEQ ID N°36, ou la séquence SEQ ID N°36 dans laquelle une ou deux mutations ponctuelles peuvent exister.

**13.** Procédé de détermination du profil quantitatif et qualitatif selon la revendication 6 ou 8, **caractérisé en ce que**, quand le type donné de la chaîne lourde d'immunoglobuline est une chaîne lourde d'IgE et quand les produits d'amplifications séparées obtenus pour chacun des sous-groupes VH sont encore élongués, la sonde antisens marquée CH a la séquence SEQ ID N°37, ou la séquence SEQ ID N°37 dans laquelle une à trois mutations ponctuelles peuvent exister, sauf pour les nucléotides 1 à 6 de sa partie 3'.

**14.** Procédé de détermination du profil quantitatif et qualitatif selon la revendication 9,
**caractérisé en ce que**, quand le type donné de la chaîne lourde d'immunoglobuline est une chaîne lourde d'IgG et quand les amplifications séparées sont des amplifications séparées en temps réel, la sonde d'hydrolyse marquée CH a la séquence SEQ ID N°34, ou la séquence SEQ ID N°34 dans laquelle une ou deux mutations ponctuelles peuvent exister.

**15.** Procédé de détermination du profil quantitatif et qualitatif selon la revendication 9, **caractérisé en ce que**, quand le type donné de la chaîne lourde d'immunoglobuline est une chaîne lourde d'IgG et quand les produits d'amplifications séparées obtenus pour chacun des sous-groupes VH sont encore élongués, la sonde antisens marquée CH a la séquence SEQ ID N°35, ou la séquence SEQ ID N°35 dans laquelle une à trois mutations ponctuelles peuvent exister, sauf pour les nucléotides 1 à 6 de sa partie 3'.

**16.** Procédé de détermination du profil quantitatif et qualitatif selon l'une quelconque des revendications 2 à 15, **caractérisé en ce que** d'autres amplifications séparées pour chacun des sous-groupes JH sont réalisées à partir des produits d'amplifications séparées obtenus pour au moins un sous-groupe VH donné de sous-groupes VH portant l'amorce antisens CH,
lesdites autres amplifications séparées étant réalisées en utilisant une amorce sens interne VH correspondant au sous-groupe VH donné, et associées à un ensemble d'amorces antisens JH correspondant aux sous-groupes JH et susceptibles d'hybridation spécifique dans des conditions stringentes avec les acides nucléiques codant les segments de jonction du type donné de chaîne lourde d'immunoglobuline.

**17.** Procédé de détermination du profil quantitatif et qualitatif selon la revendication 16, **caractérisé en ce que** les autres amplifications séparées sont des amplifications en temps réel réalisées en utilisant une sonde sens marquée VH, de préférence une sonde d'hydrolyse sens marquée VH, susceptible d'hybridation spécifique dans des conditions stringentes avec le segment variable du type donné de chaîne lourde d'immunoglobuline et susceptible d'émettre un signal détectable à chaque fois qu'un cycle d'amplification se produit, et **caractérisé en ce que** le signal obtenu pour chaque sous-groupe JH est mesuré.

**18.** Procédé de détermination du profil quantitatif et qualitatif selon la revendication 16 ou 17, **caractérisé en ce que**, quand le sous-groupe VH donné est le sous-groupe VH5, l'amorce sens interne VH5 a la séquence SEQ ID N° 31, ou la séquence SEQ ID N° 31
dans laquelle une à trois mutations ponctuelles peuvent exister, sauf pour les nucléotides 1 à 6 de sa partie 3'.

**19.** Procédé de détermination du profil quantitatif et qualitatif selon la revendication 18, **caractérisé en ce que** la sonde d'hydrolyse sens marquée VH a la séquence SEQ ID N°32, ou la séquence SEQ ID N°32 dans laquelle une ou deux mutations ponctuelles peuvent exister.

**20.** Procédé de détermination du profil quantitatif et qualitatif selon l'une quelconque des revendications 2 à 15, **caractérisé en ce que** les élongations séparées sont réalisées pour chacun des sous-groupes JH à partir des produits d'amplifications séparées obtenus pour au moins un sous-groupe VH donné des sous-groupes VH portant l'amorce antisens CH,
lesdites autres élongations séparées étant réalisées en utilisant un ensemble d'amorces antisens marquées JH correspondant aux sous-groupes JH et susceptibles d'hybridation spécifique dans des conditions stringentes avec les acides nucléiques codant les segments de jonction du type donné de chaîne lourde d'immunoglobuline, lesdites amorces antisens marquées JH étant susceptibles d'émettre un signal détectable,
et **caractérisé en ce que** les produits d'élongation sont séparés, pour chacun des sous-groupes JH, par rapport à leur longueur, le signal obtenu pour les produits d'allongement séparés est mesuré, et le profil quantitatif et qualitatif de l'intensité de marquage relatif à la longueur du produit d'élongation est établi, pour chacun des sous-groupes JH pour le sous-groupe VH donné.

**21.** Procédé de détermination du profil quantitatif et qualitatif selon l'une quelconque des revendications 16 à 20, **caractérisé en ce que** l'ensemble des amorces sens JH, éventuellement marquées, comprend au moins les 6 sous-groupes suivants d'amorces JH correspondant aux sous-groupes JH :

- l'amorce JH1 ayant la séquence SEQ ID N°16, et
- l'amorce JH2 ayant la séquence SEQ ID N°17, et
- l'amorce JH3 ayant la séquence SEQ ID N°18, et
- les amorces JH4 ayant les séquences SEQ ID N° 19 à SEQ ID N°21, et
- l'amorce JH5 ayant la séquence SEQ ID N°22, et
- les amorces JH6 ayant les séquences SEQ ID N°23 à SEQ ID N°25.

**22.** Procédé de détermination du profil quantitatif et qualitatif selon la revendication 21, **caractérisé en ce que** les séquences SEQ ID N°16 à SEQ ID N°25 peuvent contenir au moins une à trois mutations ponctuelles, sauf pour les nucléotides 1 à 6 de leur partie 3'.

**23.** Ensemble d'amorces sens VH susceptibles d'hybridation spécifique dans des conditions stringentes avec les acides nucléiques codant les segments variables (VH) des chaînes lourdes d'immunoglobuline, lesdits segments variables étant distribués entre au moins 8 sous-groupes VH, associés à une amorce inverse CH, ou un mélange de ceux-ci, susceptibles d'hybridation spécifique dans des conditions stringentes avec l'acide nucléique codant le segment constant (CH) d'un type donné d'une chaîne lourde d'immunoglobuline, **caractérisé en ce que** l'ensemble d'amorces sens VH comprend au moins 8 sous-groupes suivants des amorces VH correspondant aux sous-groupes VH :

- les amorces VH1 ayant les séquences SEQ ID N°1 à SEQ ID N°3, et
- l'amorce VH2 ayant la séquence SEQ ID N°4, et
- les amorces VH3a ayant les séquences SEQ ID N°5 et SEQ ID N°6, et
- les amorces VH3b ayant les séquences SEQ ID N° 7 à SEQ ID N°10, et
- les amorces VH4 ayant les séquences SEQ ID N°11 et SEQ ID N°12, et
- l'amorce VH5 ayant la séquence SEQ ID N°13, et
- l'amorce VH6 ayant la séquence SEQ ID N°14, et
- l'amorce VH7 ayant la séquence SEQ ID N°15.

**24.** Ensemble d'amorces sens VH selon la revendication 23, **caractérisé en ce que** les séquences SEQ ID N°1 à SEQ ID N°15 peuvent contenir au moins une à trois mutations ponctuelles, sauf pour les nucléotides 1 à 6 de leur partie 3'.

**25.** Ensemble d'amorces sens VH selon la revendication 23 ou 24, **caractérisé en ce que** l'amorce antisens CH est choisie parmi les amorces antisens CH susceptibles d'hybridation spécifique dans des conditions stringentes avec les acides nucléiques codant les segments constants (CH) de la chaîne lourde d'IgM, la chaîne lourde d'IgE et la chaîne lourde d'IgA.

**26.** Ensemble d'amorces sens VH selon la revendication 25, **caractérisé en ce que**, quand l'amorce antisens CH est susceptible d'hybridation spécifique dans des conditions stringentes avec l'acide nucléique codant le segment constant (CH) de la chaîne lourde d'IgM, l'amorce antisens CH a la séquence SEQ ID N°26, ou la séquence SEQ ID N°26 dans laquelle une à trois mutations ponctuelles peuvent exister, sauf pour les nucléotides 1 à 6 de sa partie 3'.

**27.** Ensemble d'amorces sens VH selon la revendication 25, **caractérisé en ce que**, quand l'amorce antisens CH est susceptible d'hybridation spécifique dans des conditions stringentes avec l'acide nucléique codant le segment constant (CH) de la chaîne lourde d'IgE, l'amorce antisens CH a la séquence SEQ ID N°33, ou la séquence SEQ ID N°33 dans laquelle une à trois mutations ponctuelles peuvent exister, sauf pour les nucléotides 1 à 6 de a partie 3'.

**28.** Ensemble d'amorces sens VH selon la revendication 23 ou 24, **caractérisé en ce que**, quand le type donné de la chaîne lourde d'immunoglobuline est le type IgG, un mélange de deux amorces antisens CH est associé à l'ensemble des amorces sens VH,

lesdites deux amorces antisens CH ayant les séquences SEQ ID N°27 et SEQ ID N°28, ou les séquences SEQ ID N°27 et SEQ ID N°28 dans lesquelles une à trois mutations ponctuelles peuvent exister, sauf pour les nucléotides 1 à 6 de sa partie 3'.

**29.** Procédé de diagnostic *in vitro* d'un état choisi dans le groupe constitué par une maladie auto-immune, un lymphome des cellules B, une maladie immunodépressive, et un état qui résulte d'une transplantation de moelle osseuse, à partir d'un test vaccinal ou d'une réaction allergique chez un sujet, **caractérisé en ce qu'**il comprend les étapes suivantes consistant à :

(1) déterminer le profil quantitatif et qualitatif du répertoire d'un type donné de la chaîne lourde d'immunoglobuline provenant d'un échantillon de tissu dudit sujet, dans lequel ladite détermination comprend les opérations consistant à :

(a) obtenir soit l'ADNc provenant de l'ARNm exprimé par l'échantillon de tissu soit l'extrait d'ADN cellulaire de l'échantillon de tissu,
(b) réaliser l'amplification de l'ADNc obtenu à l'étape (a) avec un ensemble d'amorces sens VH susceptibles d'hybridation spécifique dans des conditions stringentes avec les acides nucléiques codant les segments variables (VH) des chaînes lourdes d'immunoglobuline, lesdits segments variables étant distribués parmi des sous-groupes VH, associés à une amorce antisens CH, ou un mélange de celles-ci, susceptibles d'hybridation spécifique, dans des conditions stringentes, avec l'acide nucléique codant le segment constant (CH) d'un type donné d'une chaîne lourde d'immunoglobuline, et
(c) déterminer le profil quantitatif et qualitatif du répertoire dudit type de chaînes lourdes d'immunoglobuline pour chaque sous-groupe VH,

dans lequel l'ensemble des amorces sens VH comprend au moins les 8 sous-groupes suivants d'amorces VH correspondant aux sous-groupes VH :

- les amorces VH1 ayant les séquences SEQ ID N°1 à SEQ ID N°3, et
- l'amorce VH2 ayant la séquence SEQ ID N°4, et
- les amorces VH3a ayant les séquences SEQ ID N°5 et SEQ ID N°6, et
- les amorces VH3b ayant les séquences SEQ ID N°7 à SEQ ID N°10, et
- les amorces VH4 ayant les séquences SEQ ID N°11 et SEQ ID N°12, et
- l'amorce VH5 ayant la séquence SEQ ID N°13, et
- l'amorce VH6 ayant la séquence SEQ ID N°14, et
- l'amorce VH7 ayant la séquence SEQ ID N°15,

(2) comparer le profil quantitatif et qualitatif obtenu à l'étape (1) avec un profil témoin quantitatif et qualitatif dudit type donné de chaîne lourde d'immunoglobuline, la démonstration d'une modification significative du profil obtenu à l'étape (1) étant significative d'un tel état chez le sujet.

**30.** Procédé de suivi *in vitro* d'un traitement d'un état choisi dans le groupe constitué par une maladie auto-immune, un lymphome des cellules B, une maladie immunodépressive, et un état qui résulte d'une transplantation de moelle osseuse, à partir d'un test vaccinal ou d'une réaction allergique chez un sujet, **caractérisé en ce qu'**il comprend les étapes suivantes consistant à :

(1) éventuellement, déterminer avant le traitement le profil quantitatif et qualitatif du répertoire d'un type donné de chaîne lourde d'immunoglobuline à partir d'un échantillon de tissus dudit sujet,
(2) déterminer, au cours du traitement, le profil quantitatif et qualitatif du répertoire d'un type donné de chaîne lourde d'immunoglobuline à des moments donnés à partir d'échantillons de tissu dudit sujet,
dans lequel ladite détermination comprend les opérations consistant à :

(a) obtenir soit l'ADNc provenant de l'ARNm exprimé à partir l'échantillon de tissu soit l'extrait d'ADN cellulaire de l'échantillon de tissu,
(b) réaliser l'amplification de l'ADNc obtenu à l'étape (a) avec un ensemble d'amorces sens VH susceptibles d'hybridation spécifique dans des conditions stringentes avec les acides nucléiques codant les segments variables (VH) des chaînes lourdes d'immunoglobuline, lesdits segments variables étant distribués parmi des sous-groupes VH, associés à une amorce antisens CH, ou un mélange de ceux-ci, susceptibles d'hybridation spécifique, dans des conditions stringentes, avec l'acide nucléique codant le segment constant (CH) d'un type donné d'une chaîne lourde d'immunoglobuline, et
(c) déterminer le profil quantitatif et qualitatif du répertoire dudit type de chaînes lourdes d'immunoglobuline pour chaque sous-groupe VH,

dans lequel l'ensemble des amorces sens VH comprend au moins les 8 sous-groupes suivants d'amorces VH correspondant aux sous-groupes VH :

- les amorces VH1 ayant les séquences SEQ ID N°1 à SEQ ID N°3, et
- l'amorce VH2 ayant la séquence SEQ ID N°4, et
- les amorces VH3a ayant les séquences SEQ ID N°5 et SEQ ID N°6, et
- les amorces VH3b ayant les séquences SEQ ID N°7 à SEQ ID N°10, et
- les amorces VH4 ayant les séquences SEQ ID N°11 et SEQ ID N°12, et
- l'amorce VH5 ayant la séquence SEQ ID N°13, et
- l'amorce VH6 ayant la séquence SEQ ID N°14, et
- l'amorce VH7 ayant la séquence SEQ ID N°15,

(3) comparer les profils quantitatifs et qualitatifs obtenus à l'étape (2) et éventuellement à l'étape (1) avec les uns avec les autres et éventuellement avec un profil témoin quantitatif et qualitatif dudit type donné de chaîne lourde d'immunoglobuline, la démonstration d'une modification significative du profil obtenu à l'étape (1) étant significative d'un tel état chez le sujet.

**31.** Kit de détermination du profil quantitatif et qualitatif du répertoire d'un type donné d'une chaîne lourde d'immunoglobuline exprimée par une population de lymphocyte B présente dans un échantillon de tissus, **caractérisé en ce qu'**il comprend l'ensemble des amorces sens VH selon l'une quelconque des revendications 23 à 28 associées à l'amorce antisens CH.

**32.** Kit selon la revendication 31, **caractérisé en ce qu'**il comprend en outre un ensemble d'amorces antisens JH, éventuellement marquées, correspondant aux sous-groupes JH et susceptibles d'hybridation spécifique dans des conditions stringentes avec les acides nucléiques codant les segments de jonction du type donné de la chaîne lourde d'immunoglobuline.

**33.** Kit selon la revendication 32, **caractérisé en ce que** l'ensemble des amorces antisens JH comprend les 6 sous-groupes suivants d'amorces JH correspondant aux sous-groupes JH :

- l'amorce JH1 ayant la séquence SEQ ID N°16, et
- l'amorce JH2 ayant la séquence SEQ ID N°17, et
- l'amorce JH3 ayant la séquence SEQ ID N°18, et
- les amorces JH4 ayant les séquences SEQ ID N°19 à SEQ ID N°21, et
- l'amorce JH5 ayant la séquence SEQ ID N°22, et
- les amorces JH6 ayant les séquences SEQ ID N°23 à SEQ ID N°25.

**34.** Kit selon la revendication 33, **caractérisé en ce que** les séquences SEQ ID N°16 à SEQ ID N°25 peuvent contenir au moins une à trois mutations ponctuelles, sauf pour les nucléotides 1 à 6 de leur partie 3'.

**35.** Utilisation du kit selon l'une quelconque des revendications 31 à 34, pour le diagnostic *in vitro* d'un état choisi dans le groupe constitué par une maladie auto-immune, un lymphome des cellules B, une maladie immunodépressive, et un état qui résulte d'une transplantation de moelle osseuse, à partir d'un test vaccinal ou d'une réaction allergique chez un sujet.

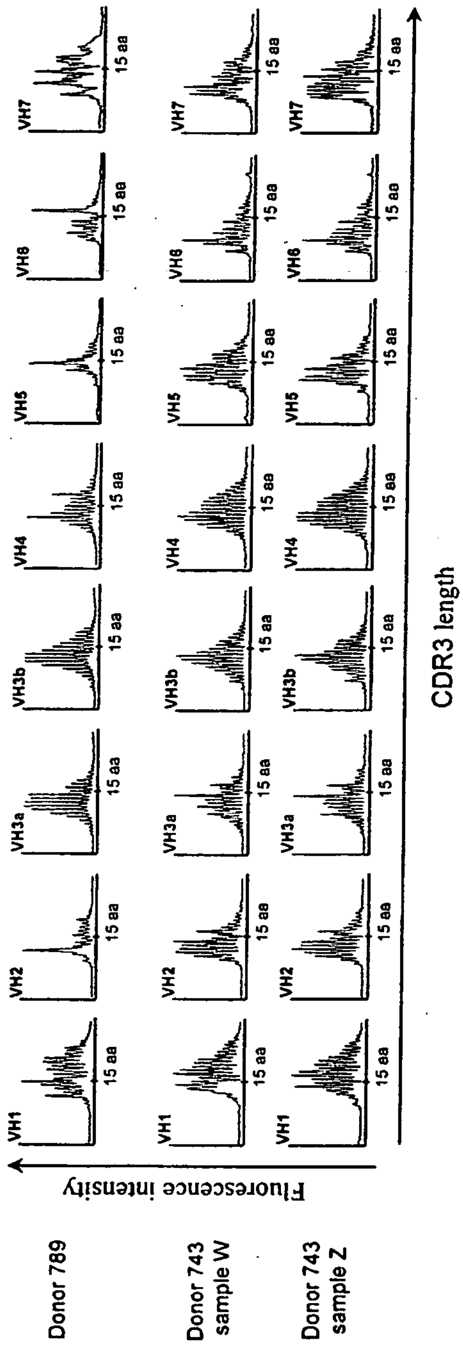


Figure 1A

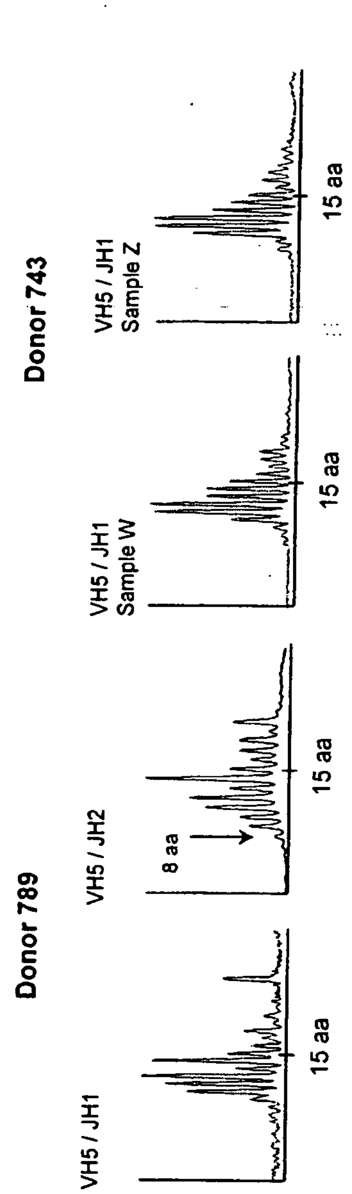


Figure 1B

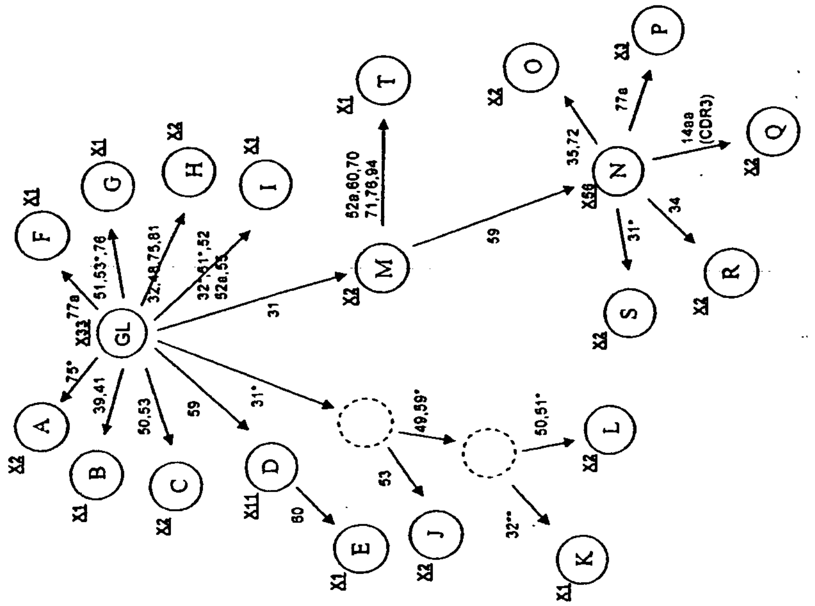

Mutations tree of clone B

Figure 2B

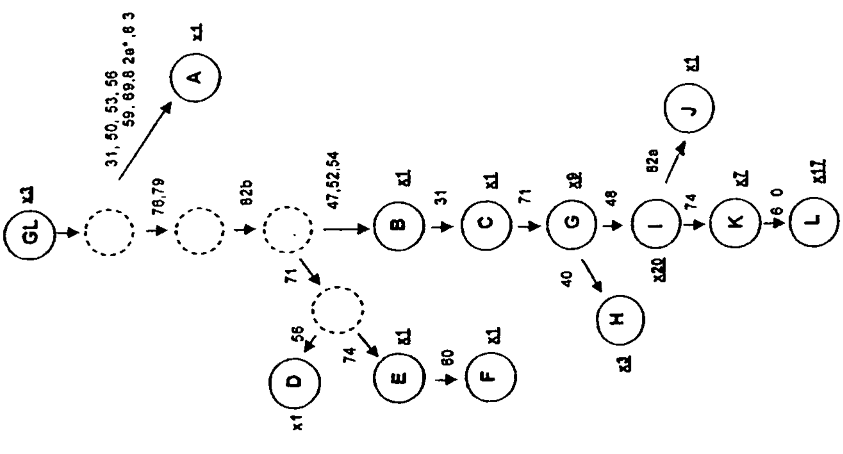

Mutations tree of clone A

Figure 2A

# Donor 743

| Donor 743 | tW-tov | tZ-tov | tov | tW+tZ | dW-dov | dZ-dov | dov | dW+dZ |
|---|---|---|---|---|---|---|---|---|
| seq. nb | 517 | 488 | 268 | 1273 | 202 | 199 | 20 | 421 |
| % | 40,6 | 38,4 | 21 | 100 | 48 | 47,3 | 4,7 | 100 |

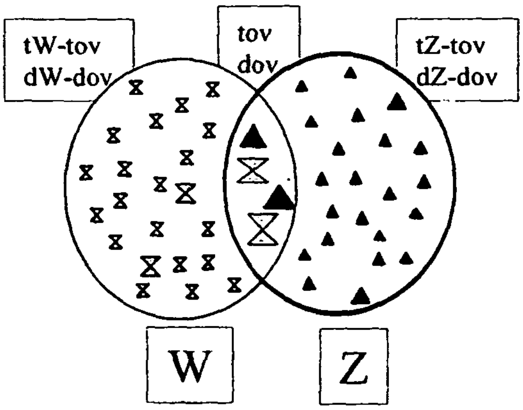


Figure 3A

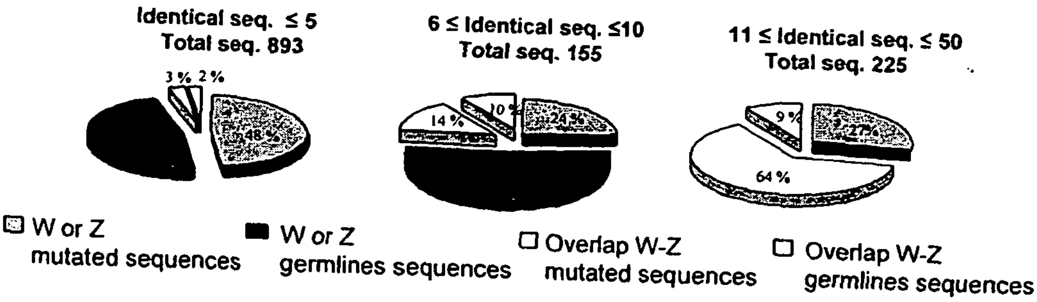


Figure 3 B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 566685 A
- EP 672184 A


### Non-patent literature cited in the description


- **SCHATZ.** *Cell,* 1989, vol. 59, 1035-48
- **OETTINGER.** *Science,* 1990, vol. 248, 1517-23
- **BOLLUM, F. J.** *Adv Enzymol Relat Areas Mol Biol,* 1978, vol. 47, 347-74
- **KOMORI.** *Science,* 1993, vol. 261, 1171-5
- **GILFILLAN.** *Science,* 1993, vol. 261, 1175-8
- **WU.** *J Clin Immunol,* 2003, vol. 23, 235-46
- **HONJO.** *Annu Rev Immunol,* 2002, vol. 20, 165-96
- **THOMPSON ; NEIMAN.** *Cell,* 1987, vol. 48, 369-78
- **REYNAUD.** *Cell,* 1987, vol. 48, 379-88
- **MURAMATSU.** *Cell,* 2000, vol. 102, 553-63
- **REVY.** *Cell,* 2000, vol. 102, 565-75
- **ARAKAWA.** *Science,* 2002, vol. 295, 1301-6
- **OKAZAKI.** *Nature,* 2002, vol. 416, 340-5
- **YOSHIKAWA.** *Science,* 2002, vol. 296, 2033-6
- **TUAILLON ; CAPRA.** *J Immunol,* 2000, vol. 164, 6387-97
- **CABANIOLS.** *J Exp Med,* 2001, vol. 194, 1385-90
- **WAGNER.** *Proc Natl Acad Sci U S A,* 1998, vol. 95, 14447-52
- **ARSTILA.** *Science,* 1999, vol. 286, 958-61
- **BARON.** *Immunity,* 2003, vol. 18, 193-204
- **GOJOBORI et al.** *Mol Biol Evol,* 1986, vol. 3, 156-67
- **SCHITTEK ; RAJEWSKY.** *J Exp Med,* 1992, vol. 176, 427-38
- **BEREK.** *Cell,* 1991, vol. 67, 1121-9
- **MACLENNAN ; GRAY.** *Immunol Rev,* 1986, vol. 91, 61-85
- **WILLIAMS.** *Immunity,* 2000, vol. 13, 409-17
- **KLEIN.** *Blood,* 1997, vol. 89, 1288-98
- **KLEIN.** *J Exp Med,* 1998, vol. 188, 1679-89
- **LOEMBÉ.** *Eur J Immunol.,* December 2002, vol. 32 (12), 3678-88
- **FAIS.** *J Clin Invest.,* 15 October 1998, vol. 102 (8), 1515-25
- **SAMBROOK et al.** Molecular Cloning: A Laboratory Manual. Cold Spring Harbor Laboratory Press, 1989
- Current Protocols in Molecular Biology. Current Protocols Press, 1994
- **BERGER ; KIMMEL.** Methods in Enzymology: Guide to Molecular Cloning Techniques. Academic Press, Inc, 1987, vol. 152
- Current Protocols in Molecular Biology. 1994
- **LIM.** *J Immunol Methods,* 2002, vol. 261, 177-94
- **LEFRANC.** *Nucleic Acids Res,* 1999, vol. 27, 209-12
- V Base Sequence Directory. **TOMLINSON.** M. C. f. P. Engineering. 1998
- **LEFRANC, M.P. et al.** *Nucleic Acids Research,* vol. 29 (1), 207-209
- **PANNETIER.** The Antigen T Cell receptor: Selected Protocols and Applications. Chapman & Hall, 1997, 287
- **PANNETIER.** *Proc Natl Acad Sci U S A,* 1993, vol. 90, 4319-23
- **CASROUGE.** *J Immunol,* 2000, vol. 164, 5782-7
- **RAAPHORST.** *Biotechniques,* 1996, vol. 84, 86-7
- **ODENDAHL.** *J Immunol,* 2000, vol. 165, 5970-9
- **DELASSUS.** *J Immunol Methods,* 1995, vol. 184, 219-29